# FASCICULE DE BREVET EUROPEEN

(11) **EP 3 568 402 B1**
(45) Date de publication et mention de la délivrance du brevet: **02.11.2022**
(21) Numéro de dépôt: 18700279.5
(22) Date de dépôt: 10.01.2018
(51) Int. Cl.: C07F 5/00, A61K 49/00, C07F 5/02, G01N 33/533

(54) **COMPLEXES DE LANTHANIDES À BASE DE DÉRIVÉS D'ACIDE TRIÉTHYLÈNETÉTRAMINE-N,N,N',N'',N''',N'''-HEXAACÉTIQUE**
LANTHANIDKOMPLEXE MIT DERIVATEN VON TRIETHYLENTETRAMIN-N,N,N',N'',N''',N'''-HEXAESSIGSÄURE
LANTHANIDE COMPLEXES COMPRISING DERIVATIVES OF TRIETHYLENETETRAMINE-N,N,N',N'',N''',N'''-HEXAACETIC ACID

(30) Priorité: 11.01.2017 FR 1750240
(43) Date de publication de la demande: 20.11.2019
(73) Titulaire: Université d'Orléans, 45100 Orléans (FR); Centre National de la Recherche Scientifique, 75016 Paris (FR)
(72) Inventeur: ELISEEVA, Svetlana, 45650 Saint Jean Le Blanc (FR); SUZENET, Franck, 45380 La Chapelle Saint Mesmin (FR); ROUTIER, Sylvain, 45510 Tigy (FR); BEN OTHMAN, Raja, 94430 Chennevières-sur-Marne (FR); COLLET, Alexandra, 45100 Orleans (FR); MARTINIC, Ivana, 21412 Pucisca (HR); DELATOUCHE, Régis, 70150 Kuopio (FI); PETOUD, Stéphane, 45160 Saint-Hilaire-Saint Mesmin (FR)
(74) Mandataire: Lavoix
(86) Numéro de dépôt international: PCT/EP2018/050517
(87) Numéro de publication internationale: WO 2018/130545

(56) Documents cités:
- WO-A1-2014/123418
- DE-A1- 19 505 960
- TAKUYA NISHIOKA ET AL: "Minocycline-Based Europium(III) Chelate Complexes: Synthesis, Luminescent Properties, and Labeling to Streptavidin", HELVETICA CHIMICA ACTA, vol. 92, no. 11, 1 novembre 2009 (2009-11-01), pages 2357-2374, XP055389281, CH ISSN: 0018-019X, DOI: 10.1002/hlca.200900175
- Kyle Gee: "SITE-SPECIFIC LABELING OF AFFINITY TAGS IN FUSION PROTEINS", US2016025713 A1, 1 janvier 2016 (2016-01-01), pages 1-2, XP055389203, Extrait de l'Internet: URL:www [extrait le 2017-07-10] -& WO 2004/025259 A2 (INVITROGEN CORP) 25 mars 2004 (2004-03-25)
- Yu-Dong Xiao ET AL: "MRI contrast agents: Classification and application (Review)", INTERNATIONAL JOURNAL OF MOLECULAR MEDICINE, vol. 38, no. 5, 21 November 2016 (2016-11-21), pages 1319-1326, XP055688986, GR ISSN: 1107-3756, DOI: 10.3892/ijmm.2016.2744
- Anonymous: "Covalent bond - Wikipedia", Wikipedia, 7 September 2020 (2020-09-07), pages 1-9, XP055729776, Retrieved from the Internet: URL:https://en.wikipedia.org/wiki/Covalent _bond [retrieved on 2020-09-11]
- Anonymous: "IUPAC - covalent bond (C01384)", , 24 February 2014 (2014-02-24), pages 1-2, XP055729778, Retrieved from the Internet: URL:https://goldbook.iupac.org/terms/view/ C01384 [retrieved on 2020-09-11]
- KUMARI SHIKHA ET AL: "Amide Bond Bioisosteres: Strategies, Synthesis, and Successes", JOURNAL OF MEDICINAL CHEMISTRY, vol. 63, no. 21, 20 July 2020 (2020-07-20) , pages 12290-12358, XP055816341, ISSN: 0022-2623, DOI: 10.1021/acs.jmedchem.0c00530

## Description

La présente invention a pour objet des complexes de lanthanides à base de dérivés d'acide triéthylènetétramine-N,N,N',N",N‴,N‴-hexaacétique. En particulier, la présente invention a pour objet des composés dérivés d'acide triéthylènetétramine-N,N,N',N",N‴,N‴-hexaacétique, greffés à une antenne, pouvant se complexer aux lanthanides.

Les méthodes liées à la fluorescence (microscopie et macroscopie) présentent les avantages d'être très sensibles, de permettre des mesures en temps réel, peu dangereuses pour les milieux biologiques (en raison de la faible quantité d'agents d'imagerie nécessaires à la condition d'utiliser des longueurs d'onde d'excitation adaptées) et très accessibles (en terme de coûts de fabrication et d'utilisation), de temps expérimentaux, de mobilité et de niveau de spécialisation des utilisateurs.

La grande majorité des fluorophores organiques commerciaux sont fortement photosensibles et se dégradent en présence de lumière d'excitation.

Les rapporteurs fluorescents basés sur des nanocristaux semi-conducteurs constituent une autre gamme de choix d'agents d'imagerie optique. Une des limites d'utilisation de ces systèmes est la taille relativement élevée de ces nanoparticules qui peuvent interférer avec les structures et les fonctions de la cellule. Un autre facteur limitant est la forte toxicité des métaux qui constituent ces nanoparticules (cadmium, tellure et sélénium) dans le cas où dernières se dissocient.

La famille des lanthanides inclut 14 éléments aux propriétés optiques extrêmement intéressantes et uniques, caractérisées par des bandes d'émissions précises et étroites, allant du visible au proche infrarouge (> 1 200 nm). Chaque lanthanide possède des propriétés spectrales distinctes et identifiables. Il est ainsi possible de décliner, sur la base d'une même technologie, toute une gamme de longueurs d'ondes différentes par le simple choix de la nature du lanthanide à incorporer dans la molécule. Ces bandes d'émission sont beaucoup plus étroites que celle des fluorophores organiques et des nanoparticules fluorescentes (quantum dots), ce qui autorise une meilleure discrimination spectrale et des essais multiplexes. De plus, la position (en nm) de ces bandes d'émission ne varie pas en fonction de l'environnement (cellulaire, pH, températures, sites hydrophiles/hydrophobes...) ce qui facilite leurs détections et minimise l'adaptation de l'équipement (filtre unique pour un lanthanide donné).

L'article de Nishioka et al. (Helvetica Chimica Acta, Vol.92, no.11, 2009, pages 2357-2374) concerne des complexes comprenant de l'europium et à base de minocycline.

WO 2004/025259 concerne des composés fluorescents comprenant un motif BODIPY.

DE 195 05 960 concerne des conjugués pouvant comprendre un composé métallique et notamment du gadolinium, et leurs utilisations pour le dosage de médicaments ou pour le diagnostic.

WO 2014/123418 décrit un système comprenant une cyanine pour se lier aux cellules nécrotiques, et comprenant en outre (a) un agent choisi parmi les composés pour l'imagerie, les composés thérapeutiques et un facteur de terminaison ; (b) un véhicule pour le transport dudit agent ; et (c) une molécule non réactive avec le corps et étant capable d'interagir avec une contrepartie.

Yu-Dong Xiao et al. (International Journal of Molecular Medecine, vol.38, no.5, 2016, pages 1319-1326) décrit des agents de contraste pour IRM. Cet article cite notamment les lanthanides, et plus particulièrement le gadolinium.

En dépit de la forte demande des chercheurs en biologie et des médecins, il n'existe cependant à ce jour aucune sonde fluorescente à base de lanthanide qui soit excitable et émettrice au-dessus de 650 nm, correspondant ainsi à la fenêtre biologique. Globalement, les sondes proches infrarouges actuelles sont de nature organique, les commerciales sont peu nombreuses et souffrent de limitations comme la tendance au photoblanchiment et des déplacements de Stokes restreints.

La présente invention a donc pour but de fournir un complexe de lanthanides, de taille réduite, émettant dans le proche infrarouge et pouvant être excité dans le proche infrarouge.

Un autre but de l'invention consiste à fournir un système luminescent émettant et absorbant dans le proche infrarouge et permettant d'observer divers systèmes biologiques sans les détruire.

Un autre but de l'invention consiste à fournir un système luminescent interagissant peu voire pas du tout avec les matières biologiques, et donc limitant les interférences avec le métabolisme normal des systèmes biologiques.

La présente demande décrit un complexe comprenant au moins un lanthanide (Ln) et au moins un composé (C) comprenant un motif de formule (I) suivante : 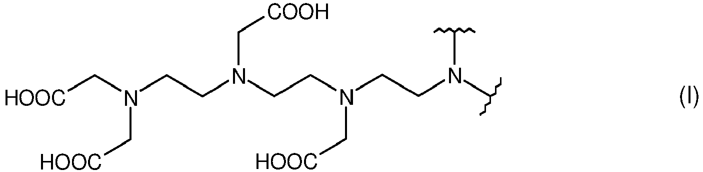 ledit motif de formule (I) étant relié de façon covalente à au moins une antenne qui absorbe à une longueur d'onde allant de 500 nm à 900 nm.

La présente demande décrit également un complexe comprenant au moins un lanthanide (Ln) et au moins un composé (C) répondant à la formule (II) suivante : 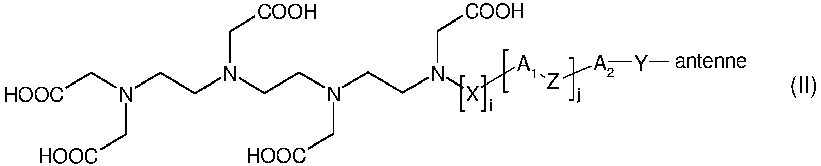 dans laquelle :
- i est 0 ou 1 ;
- j est un nombre entier compris entre 1 et 3 ;
- X est choisi parmi les fonctions -NH-NH-CO-, thioester, ester et amide,
- A₁ et A₂ sont choisis, indépendamment l'un de l'autre, parmi les radicaux (cyclo)alkylènes, linéaires ou ramifiés, comprenant de 1 à 12 atomes de carbone ;
- Z est choisi parmi les groupes -O-, -NH-, -S-, amide, ester, triazole, amine, urée, thiourée, imine, oxyme, hydrazone, sulfonamide, carbamate, amidine, phosphoramidate, disulfure et sulfonyle ; et
- Y est choisi parmi les groupes -O-, -NH-, -S-, alkylène, amide, ester, triazole, amine, urée, thiourée, imine, oxyme, hydrazone, sulfonamide, carbamate, amidine, phosphoramidate, disulfure et sulfonyle ;
ladite antenne étant choisie dans le groupe constitué des anthraquinones, des cyanines, notamment des cyanines 5 et des cyanines 7, des aza-BODIPY, des pérylènediimides et des sels de phénothiazine.

La présente invention concerne un complexe comprenant au moins un lanthanide (Ln) et au moins un composé (C) répondant à la formule (II) susmentionnée,
dans laquelle :
- i est 0 ;
- j est un nombre entier compris entre 1 et 3 ;
- X est choisi parmi les fonctions -NH-NH-CO-, thioester, ester et amide,
- A₁ et A₂ sont choisis, indépendamment l'un de l'autre, parmi les radicaux (cyclo)alkylènes, linéaires ou ramifiés, comprenant de 1 à 12 atomes de carbone ;
- Z est -C(=O)-NH- et -NH-C(=O)- ; et
- Y est choisi parmi les groupes -O-, -NH-, -S-, alkylène, amide, ester, triazole, amine, urée, thiourée, imine, oxyme, hydrazone, sulfonamide, carbamate, amidine, phosphoramidate, disulfure et sulfonyle ;
ledit motif de formule (I) étant relié de façon covalente à au moins une antenne qui absorbe à une longueur d'onde allant de 500 nm à 900 nm, ladite antenne étant choisie dans le groupe constitué des anthraquinones, des cyanines, notamment des cyanines 5 et des cyanines 7, des aza-BODIPY, des pérylènediimides et des sels de phénothiazine.

Les composés luminescents contenant des lanthanides possèdent, parmi d'autres avantages, une spécificité spectrale dans le visible et le proche-infrarouge permettant une discrimination spectrale de par leurs bandes d'émission étroites spécifiques à la nature de chaque lanthanide. Afin d'obtenir une bonne intensité de luminescence, il est important d'introduire des groupes fonctionnels sur la molécule qui la rendent capable d'absorber une grande quantité rayonnement lumineux et de transférer l'énergie résultante sur le lanthanide luminescent permettant d'obtenir l'émission d'un rayonnement de luminescence par retour du lanthanide à l'état fondamental.

Le proche infrarouge offre un grand nombre d'avantages uniques. Il permet de s'affranchir de l'auto-fluorescence des tissus, et de ce fait, permet d'améliorer le rapport signal/bruit et ainsi la sensibilité de détection. Par ailleurs, les tissus biologiques n'absorbent pas ou peu entre 640 nm et 1100 nm (fenêtre de transparence biologique), ce qui permet une excitation des sondes en profondeurs et une observation biologique non invasif (diagnostic et recherche).

En outre, les complexes de lanthanide sont photostables. Cette propriété de photostabilité est cruciale en permettant à l'agent d'imagerie d'être excité i) sur des temps expérimentaux longs et/ou ii) lors d'expériences successives et/ou iii) par des sources d'excitation puissantes (lasers pour microscopie confocale par exemple). Une quantité de photons plus importante peut ainsi être collectée sans perturber ou endommager le fonctionnement du système biologique à étudier (il est important de rappeler ici que la longueur d'onde d'excitation (> 650 nm) interagit extrêmement faiblement avec les fluides et tissus biologiques) et ainsi accroitre l'intensité et la qualité du signal collecté.

Les complexes selon l'invention peuvent être assimilés à des sondes possédant un groupe chélatant de type TTHA. De telles sondes ont l'avantage d'être de plus petite taille avec un lanthanide par antenne. Cette taille réduite permet des études cellulaires avec la minimisation des risques d'interférences avec les systèmes biologiques (cellulaires et organismes entiers - pas d'interférence avec le métabolisme normal). La souplesse de synthèse et la versatilité dans la fonctionnalisation de ces sondes, notamment avec des vecteurs biologiques, ouvrent la voie à de nombreuses applications et notamment l'association à de nombreux vecteurs biologiques. L'excitation à des longueurs d'onde supérieures à 650 nm minimise le risque de perturbation du système à étudier par la lumière d'excitation.

La présente invention concerne donc une entité obtenue par complexation entre un composé (C) tel que défini ci-dessus et au moins un lanthanide. Les complexes ainsi obtenus sont des molécules luminescentes de petite taille, notamment ayant une masse molaire inférieure à 4 kDa.

Les lanthanides sont coordinés au TTHA via les atomes d'oxygène et d'azote avec un nombre de coordination pouvant se situer entre 7 et 10. Ainsi la sphère de coordination du lanthanide est complétée et le métal est protégé de son environnement, en particulier des molécules solvants.

Les complexes selon l'invention sont obtenus par mise en présence d'un composé (C) avec une solution de lanthanides. Le composé (C) est dissous dans une solution tamponnée à pH=8, une solution de sel de lanthanide est ajoutée à la solution contenant le composé (C) dans un rapport un pour un.

Le composé (C) comprend un motif de formule (I), ledit motif étant dérivé de l'acide triéthylènetétramine-N,N,N',N",N‴,N‴-hexaacétique (TTHA).

Selon un mode de réalisation, dans le composé (C), le motif de formule (I) est relié par l'intermédiaire d'un bras à une antenne telle que définie ci-dessus.

Les composés (C) selon la présente demande peuvent être par exemple représentés par la formule générale suivante :

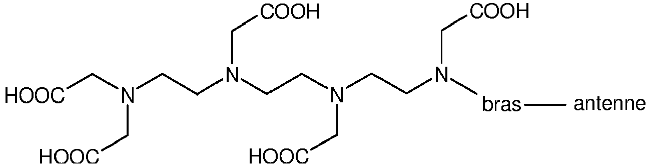

Selon l'invention, le terme "antenne" désigne une entité capable d'absorber une grande quantité de lumière d'excitation de transférer l'énergie correspondante aux lanthanides.

Selon l'invention, l'antenne est choisie dans le groupe constitué des anthraquinones, des cyanines, notamment des cyanines 5, cyanines 5,5 et des cyanines 7, des aza-BODIPY, des pérylènediimides, des sels de phénothiazine.

Le terme "bras" désigne une entité permettant de relier de façon covalente le motif de formule (I) et l'antenne.

Selon un mode de réalisation, l'antenne est reliée au motif de formule (I) de façon covalente par l'intermédiaire d'un bras répondant à la formule (B) suivante :

-(X)ᵢ-[A₁-Z]ⱼA₂-Y- (B)

dans laquelle :
- i est 0 ou 1 ;
- j est un nombre entier compris entre 1 et 3 ;
- X est choisi parmi les fonctions -NH-NH-CO-, thioester, ester et amide, de préférence parmi les fonctions ester et amide,
- A₁ et A₂ sont choisis, indépendamment l'un de l'autre, parmi les radicaux (cyclo)alkylènes, linéaires ou ramifiés, comprenant de 1 à 12 atomes de carbone ;
- Z est choisi parmi les groupes -O-, -NH-, -S-, amide, ester, triazole, amine, urée, thiourée, imine, oxyme, hydrazone, sulfonamide, carbamate, amidine, phosphoramidate, disulfure et sulfonyle ; et
- Y est choisi parmi les groupes -O-, -NH-, -S-, alkylène, amide, ester, triazole, amine, urée, thiourée, imine, oxyme, hydrazone, sulfonamide, carbamate, amidine, phosphoramidate, disulfure et sulfonyle.

Lorsque Z (ou Y) est une fonction amide, Z (ou Y) peut représenter une fonction -NH-CO- ou -CO-NH-.Lorsque Z (ou Y) est une fonction ester, Z (ou Y) peut représenter une fonction -CO-O- ou -O-CO-.

Selon un mode de réalisation, Z (ou Y) peut représenter le groupe triazole suivant :

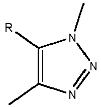

R représentant un groupe (C₁-C₆)alkyle ou (C₆-C₁₀)aryle.

Lorsque Z (ou Y) est une fonction amine, Z (ou Y) peut représenter une fonction -NH- ou -N(R)-, R représentant un groupe (C₁-C₆)alkyle ou (C₆-C₁₀)aryle.

Lorsque Z (ou Y) est une fonction urée, Z (ou Y) peut représenter une fonction -NH-CO-NH-.

Lorsque Z (ou Y) est une fonction thiourée, Z (ou Y) peut représenter une fonction -NH-CS-NH-.

Lorsque Z (ou Y) est une fonction imine, Z (ou Y) peut représenter une fonction -C(R)=N- ou -N=C(R)-, R représentant un groupe (C₁-C₆)alkyle ou (C₆-C₁₀)aryle.

Lorsque Z (ou Y) est une fonction oxyme, Z (ou Y) peut représenter une fonction -O-N=C(R)- ou -C(R)=N-O-, R représentant un groupe (C₁-C₆)alkyle ou (C₆-C₁₀)aryle.

Lorsque Z (ou Y) est une fonction hydrazone, Z (ou Y) peut représenter une fonction -NH-N=C(R)- ou -C(R)=N-NH-, R représentant un groupe (C₁-C₆)alkyle ou (C₆-C₁₀)aryle.

Lorsque Z (ou Y) est une fonction sulfonamide, Z (ou Y) peut représenter une fonction -NH-SO₂- ou -SO₂-NH-.

Lorsque Z (ou Y) est une fonction carbamate, Z (ou Y) peut représenter une fonction -NH-CO-O- ou -O-CO-NH-.

Lorsque Z (ou Y) est une fonction amidine, Z (ou Y) peut représenter une fonction -NH-C(=NH)- ou -C(=NH)-NH-.

Lorsque Z (ou Y) est une fonction phosphoramidate, Z (ou Y) peut représenter une fonction -O-P(=O)OH-NH-.

Lorsque Z (ou Y) est une fonction disulfure, Z (ou Y) peut représenter une fonction -S-S-.

Lorsque Z (ou Y) est une fonction sulfonyle, Z (ou Y) peut représenter une fonction -SO₂-.

Dans la formule (B) telle que définie ci-dessus, c'est par l'intermédiaire du radical Y que le bras se lie à l'antenne et c'est par l'intermédiaire de X, ou de A₁ lorsque i=0, que le bras se lie au motif de formule (I) dérivé de TTHA.

De préférence, dans la formule (B), X est choisi dans le groupe constitué des groupes -C(=O)-O-, -C(=O)-S-, -O-C(=O)-, -NH-C(=O)-, -N(Alk)-C(=O)-, -C(=O)-NH-, -NH-NH-C(=O)-, et -C(=O)-N(Alk)-, Alk représentant un groupe alkyle comprenant de 1 à 6 atomes de carbone.

De préférence, dans la formule (B), i=0 et Z=-C(=O)-NH-.

De préférence, dans la formule (B), A₁ et A₂ sont choisis, indépendamment l'un de l'autre, parmi les radicaux alkylènes, linéaires ou ramifiés, comprenant de 1 à 4 atomes de carbone.

De préférence, dans la formule (B), Z est choisi dans le groupe constitué des groupes -O-, -NH-, -S-, -C(=O)-O-, -O-C(=O)-, -NH-C(=O)-, -N(Alk)-C(=O)-, -C(=O)-NH- et -C(=O)-N(Alk)-, Alk représentant un groupe alkyle comprenant de 1 à 6 atomes de carbone.

Selon l'invention, le composé (C) répond à la formule (II) suivante : 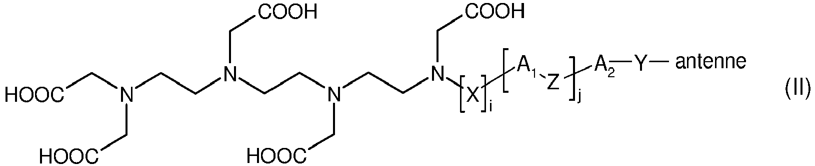 dans laquelle i, j, A₁, A₂, X, Y et Z sont tels que définis dans les revendications.

Parmi les composés (C) préférés selon l'invention, on peut citer les composés dont l'antenne est de la famille des anthraquinones.

La présente demande décrit également des complexes dans lesquels le composé (C) répond à la formule (II-1) suivante : 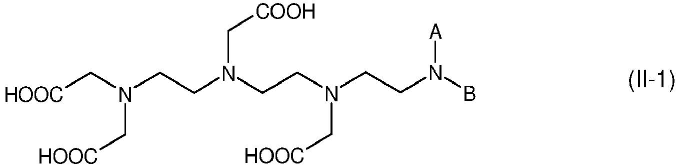 dans laquelle :
- soit A = -CH₂-COOH et B = -(X)ᵢ-[A₁-Z]ⱼ-A₂-Y-AQ, AQ représentant un motif anthraquinonique et i, j, A₁, A₂, X, Y et Z étant tels que définis dans la formule (B) ci-dessus ;
- soit A et B forment ensemble avec l'atome d'azote qui les porte un cycle incluant un motif anthraquinonique.

Selon l'invention, le terme « motif anthraquinonique » désigne un groupement dérivé de l'anthraquinone. Il désigne donc une structure polycyclique aromatique dérivée de l'anthraquinone de formule ci-dessous :

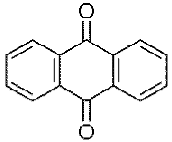

Ce motif peut donc correspondre à la structure ci-dessus comprenant un ou plusieurs substituants tels que des groupes OH, NH₂, NH(Alk), N(Alk)₂ ou alcoxy, ou encore des groupes -NH-alkylène-NH₂.

Selon un mode de réalisation, le composé (C) répond à la formule (II-1) suivante : 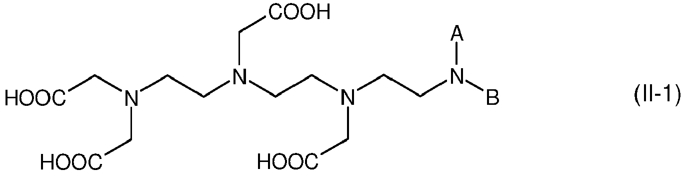 dans laquelle :
- soit A = -CH₂-COOH et B = -CH₂-C(O)-NH-(CH₂)₂-NH-AQ, AQ représentant un motif anthraquinonique ;
- soit A et B forment ensemble avec l'atome d'azote qui les porte un cycle incluant un motif anthraquinonique.

Selon un mode de réalisation, le composé (C) répond à la formule (III) suivante :

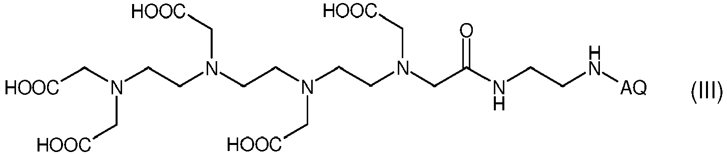

AQ représentant un motif anthraquinonique.

Selon un mode de réalisation, le composé (C) répond à la formule (IV) suivante : 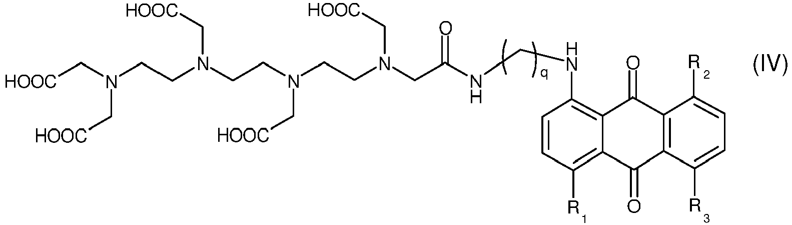
- q étant un nombre entier compris entre 1 et 10 ;
- R₁ représentant :
   - H ;
   - un groupe -NH-(CH₂)ₙ-NH₂, n représentant un nombre entier compris entre 1 et 6 ;
   - un groupe -NH-(CH₂)ₙ-R_{α}, n étant tel que défini ci-dessus et R_{α} représentant un groupe choisi parmi les halogènes, les acides carboxyliques, les esters de succinimide, les esters de tétrafluorophényle, les azotures d'acyle, les anhydrides, les halogénures d'acide, les acrylamides, les alcools, les aminés, les alcynes, les cyclooctines, les aminooxyacétamides, les azotures, les imidoesters, les esters de sulfonate, les halogéno-acétamides, les halogénures d'alkyle, les halogénures de sulfonyle, les hydrazines, les hydrazides, les isocyanates, les isothiocyanates, les tétrazines et les maléimides ;
   - un groupe (C₁-C₆)alkyle ;
   - un groupe ORₐ, Rₐ représentant H ou un groupe (C₁-C₆)alkyle ;
   - un groupe NRₐR_{b}, Rₐ et R_{b} représentant, identiques ou différents, H ou un groupe (C₁-C₆)alkyle ; et
- R₂ et R₃, identiques ou différents, représentant :
   - H ;
   - un groupe (C₁-C₆)alkyle ;
   - un groupe ORₐ, Rₐ représentant H ou un groupe choisi parmi les groupes (C₁-C₆)alkyle, (C₆-C₁₀)aryle et (C₅-C₁₀)hétéroaryle ;
   - un groupe -NH-(CH₂)ₙ-R_{α}, n étant tel que défini ci-dessus et R_{α} représentant étant tel que défini ci-dessus pour R₁ ; et
   - un groupe NRₐR_{b}, Rₐ et R_{b} représentant, identiques ou différents, H ou un groupe choisi parmi les groupes (C₁-C₆)alkyle, (C₆-C₁₀)aryle et (C₅-C₁₀)hétéroaryle.

De préférence, dans la formule (IV) susmentionnée, q=2.

De préférence, dans la formule (IV) susmentionnée, R₁ représente OH ou un groupe -NH-(CH₂)ₙ-NH₂, n étant tel que défini ci-dessus.

Selon un mode de réalisation, dans la formule (IV) susmentionnée, R₁ et R₂, identiques ou différents, de préférence identiques, représentent H ou OH.

La présente invention concerne également un complexe tel que défini ci-dessus, dans lequel le composé (C) répond à la formule (IV) suivante : 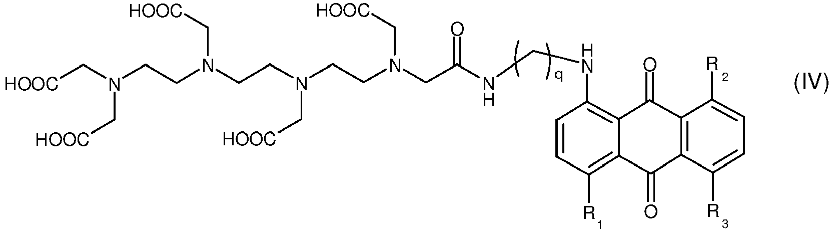 dans laquelle :
- q étant un nombre entier compris entre 1 et 6 ;
- R₁ représentant :
   - H ;
   - un groupe -NH-(CH₂)ₙ-NH₂, n représentant un nombre entier compris entre 1 et 6 ;
   - un groupe -NH-(CH₂)ₙ-R_{α}, n étant tel que défini ci-dessus et R_{α} représentant un groupe choisi parmi les halogènes, les acides carboxyliques, les esters de succinimide, les esters de tétrafluorophényle, les azotures d'acyle, les anhydrides, les halogénures d'acide, les acrylamides, les alcools, les aminés, les alcynes, les cyclooctines, les aminooxyacétamides, les azotures, les imidoesters, les esters de sulfonate, les halogéno-acétamides, les halogénures d'alkyle, les halogénures de sulfonyle, les hydrazines, les hydrazides, les isocyanates, les isothiocyanates, les tétrazines et les maléimides ;
   - un groupe (C₁-C₆)alkyle ;
   - un groupe ORₐ, Rₐ représentant H ou un groupe (C₁-C₆)alkyle ;
   - un groupe NRₐR_{b}, Rₐ et R_{b} représentant, identiques ou différents, H ou un groupe (C₁-C₆)alkyle ; et
- R₂ et R₃, identiques ou différents, représentant :
   - H ;
   - un groupe (C₁-C₆)alkyle ;
   - un groupe ORₐ, Rₐ représentant H ou un groupe choisi parmi les groupes (C₁-C₆)alkyle, (C₆-C₁₀)aryle et (C₅-C₁₀)hétéroaryle ;
   - un groupe -NH-(CH₂)ₙ-R_{α}, n et R_{α} étant tels que définis ci-dessus ; et
   - un groupe NRₐR_{b}, Rₐ et R_{b} représentant, identiques ou différents, H ou un groupe choisi parmi les groupes (C₁-C₆)alkyle, (C₆-C₁₀)aryle et (C₅-C₁₀)hétéroaryle.

De préférence, le composé (C) répond à l'une des formules suivantes :

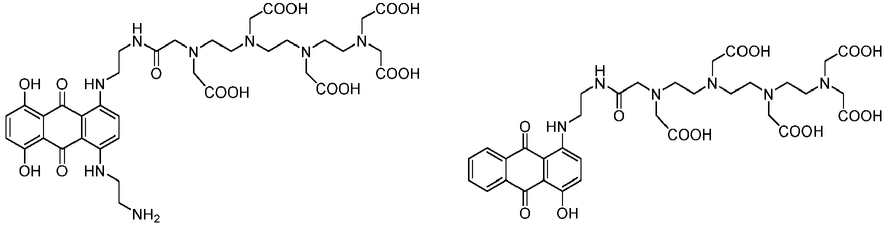

Selon un mode de réalisation, le composé (C) répond à la formule (V) suivante :

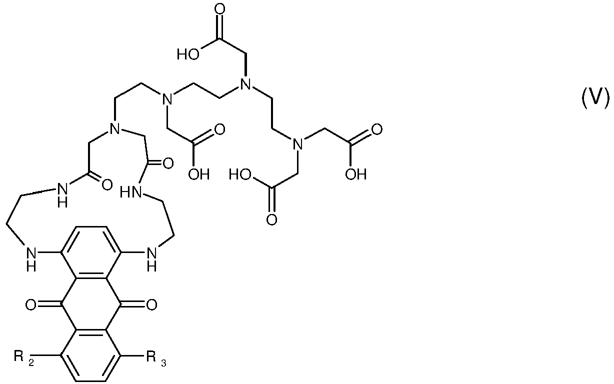

R₂ et R₃, identiques ou différents, représentant :
- H ;
- un groupe (C₁-C₆)alkyle ;
- un groupe ORₐ, Rₐ représentant H ou un groupe choisi parmi les groupes (C₁-C₆)alkyle, (C₆-C₁₀)aryle et (C₅-C₁₀)hétéroaryle ;
- un groupe NRₐR_{b}, Rₐ et R_{b} représentant, identiques ou différents, H ou un groupe choisi parmi les groupes (C₁-C₆)alkyle, (C₆-C₁₀)aryle et (C₅-C₁₀)hétéroaryle.

De préférence, le composé (C) répond à l'une des formules suivantes :

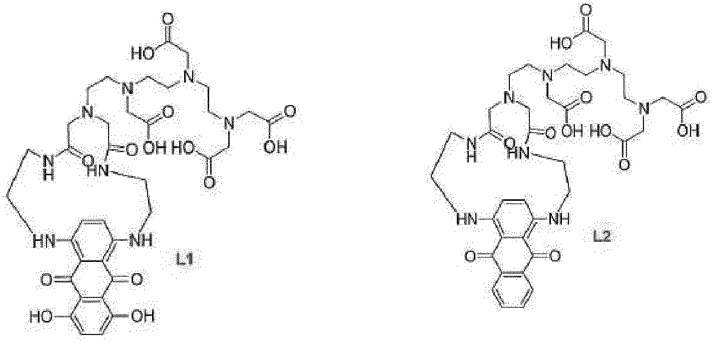

Parmi les composés (C) préférés selon l'invention, on peut citer les composés dont l'antenne est de la famille des aza-BODIPY.

Selon un mode de réalisation, le composé (C) répond à la formule (VI) suivante : 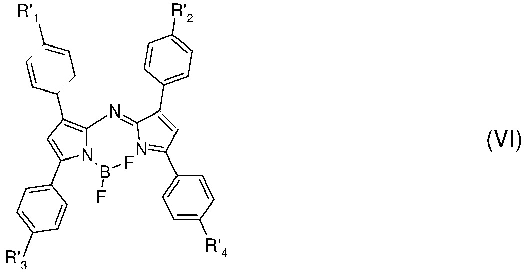 dans laquelle :
- R'₁ est H ou est choisi parmi les groupes (C₁-C₆)alkyle, (C₁-C₆)alcoxy ou NRₐR_{b}, Rₐ et R_{b}, identiques ou différents, représentant H ou un groupe (C₁-C₆)alkyle ;
- R'₂ est :
   * soit un groupe -O-A₃-COORₐ, A₃ représentant un radical (C₁-C₆)alkylène et Rₐ représentant H ou un groupe (C₁-C₆)alkyle ;
   * soit un groupe de formule (VI-1) suivante : 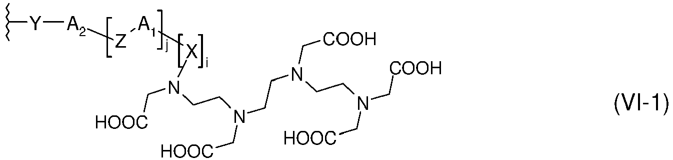 dans laquelle i, j, X, A₁, A₂, Y et Z sont tels que définis ci-dessus dans la formule (B) ;
- R'₃ est H ou un groupe de formule (VI-1) susmentionnée,
- R'₄ est H ou est choisi parmi les groupes (C₁-C₆)alkyle, (C₁-C₆)alcoxy ou NRₐR_{b}, Rₐ et R_{b}, identiques ou différents, représentant H ou un groupe (C₁-C₆)alkyle ;
sous réserve que lorsque R'₃=H alors R'₂ est un groupe de formule (VI-1) et lorsque R'₂= -O-A₃-COORₐ alors R'₃ est un groupe de formule (VI-1).

Dans la formule (VI), un groupe parmi les groupes R'₂ et R'₃ représente un groupe de formule (VI-1). En d'autres termes, le groupe R'₂ ou le groupe R'₃ représente un groupe de formule (VI-1).

Selon un mode de réalisation, dans la formule (VI), R'₂ est un groupe de formule (VI-1) et R'₃ est H. Selon un mode de réalisation, R'₂ est un groupe -O-A₃-COORₐ et R'₃ est un groupe de formule (VI-1).

Parmi les groupes de formule (VI-1) préférés, on peut citer les groupes de formule (VI-2) suivante :

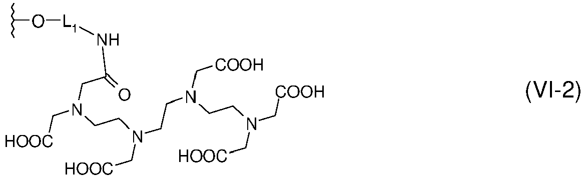

L₁ représentant un linker choisi parmi :
- les radicaux -(CH₂)ₘ-C(=O)-NH-(CH₂)ₚ-, m et p représentant un nombre entier compris entre 1 et 4, et
- les radicaux (C₁-C₆)alkylènes.

De préférence, dans la formule (VI) susmentionnée, R'₄ est H.

Selon un mode de réalisation, dans la formule (VI), R'₃ peut également être choisi parmi les groupes aryles, hétéroaryles, aminés, éthers, alkyles ou halogènes.

Selon un mode de réalisation, le composé (C) répond à la formule (VII) suivante : 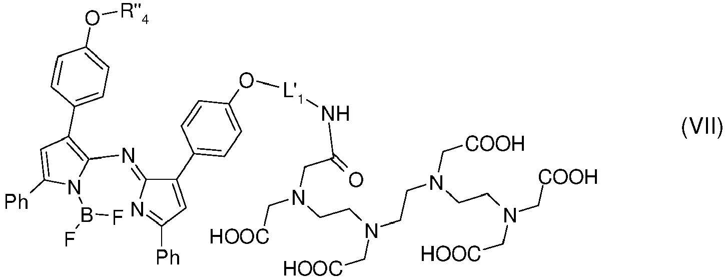 dans laquelle :
- L'₁ représente un radical -(CH₂)ₘ-C(=O)-NH-(CH₂)ₚ-, m et p représentant un nombre entier compris entre 1 et 4, et
- R"4 représente un groupe (C₁-C₆)alkyle.

Selon un mode de réalisation, le composé (C) répond à la formule (VIII) suivante : 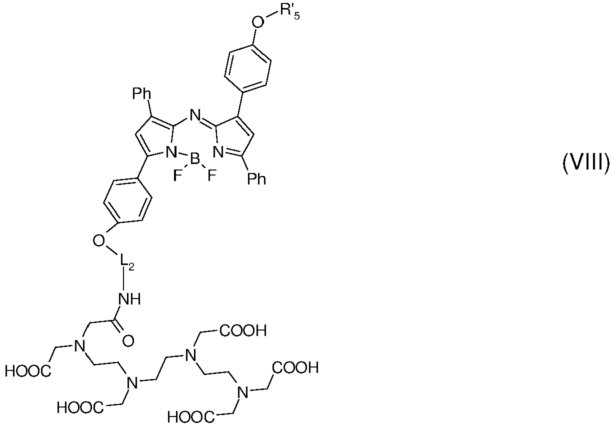 dans laquelle :
- L₂ représente un radical (C₁-C₆)alkylène, et
- R'₅ représente un groupe (C₁-C₆)alkyle, substitué par un groupe COORₐ, Rₐ représentant H ou un groupe (C₁-C₆)alkyle.

Parmi les composés préférés de formule (VI), on peut mentionner les composés suivants :

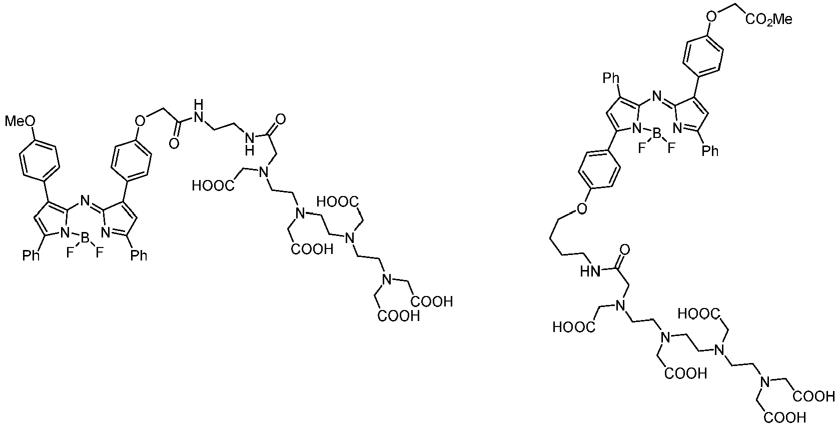

Dans le cadre de la présente invention, le terme "atome d'halogène" désigne les atomes fluor, chlore, brome ou iode.

Selon l'invention, le terme "alkyle" désigne des groupes aliphatiques hydrocarbonés, saturés, linéaires ou ramifiés comprenant, sauf mention contraire, de 1 à 6 atomes de carbone. A titre d'exemples, on peut citer les groupes méthyle, éthyle, n-propyle, isopropyle, butyle, isobutyle, tertbutyle ou pentyle.

Dans le cadre de la présente invention, on entend par "radical -(C₁-C₆)alkylène" un radical bivalent, linéaire ou ramifié, comprenant de 1 à 6 atomes de carbone, correspondant à un groupe alkyle avec un atome d'hydrogène en moins.

Selon l'invention, le terme "cycloalkyle" désigne des groupes carbonés cycliques comprenant, sauf mention contraire, de 3 à 6 atomes de carbone. A titre d'exemples, on peut citer les groupes cyclopropyle, cyclobutyle, cyclopentyle ou cyclohexyle.

Selon l'invention, le terme "alcoxy" désigne un radical -O-alkyle où le groupe alkyle est tel que précédemment défini. A titre d'exemples, on peut citer les groupes -O-(C₁-C₄)alkyle, et en particulier le groupe -O-méthyle, le groupe -O-éthyle, comme groupe -O-C₃alkyle, le groupe -O-propyle, -O-isopropyle, et comme groupe -OC₄alkyle, le groupe -O-butyle, -O-isobutyle, -O-tertbutyle.

Selon l'invention, le terme "(hétéro)aryle" englobe à la fois les termes "aryle" et "hétéroaryle", ces termes étant tels que définis ci-après.

Selon l'invention, les groupes aryles sont des groupes aromatiques cycliques comprenant entre 6 et 10 atomes de carbone. A titre d'exemples de groupes aryles, on peut citer les groupes phényle ou naphtyle.

Selon l'invention, le terme "hétéroaryle" désigne un groupe monocyclique ou bicyclique aromatique de 5 à 10 chaînons contenant de 1 à 4 hétéroatomes choisis parmi O, S ou N. Selon l'invention, le terme hétéroaryle bicyclique inclue les bicycles aromatiques fusionnés.

A titre d'exemples, on peut citer les groupes imidazolyle, thiazolyle, oxazolyle, furanyle, thiophényle, pyrazolyle, oxadiazolyle, tétrazolyle, pyridinyle, pyrazinyle, pyrimidinyle, pyridazinyle, indolyle, benzofuranyle, benzothiophényle, benzoxazolyle, benzimidazolyle, indazolyle, benzothiazolyle, isobenzothiazolyle, benzotriazolyle, quinoléinyle, isoquinoléinyle.

A titre d'hétéroaryle comprenant 5 ou 6 atomes, dont 1 à 4 atomes d'azote, on peut notamment citer les groupes représentatifs suivants : pyrrolyle, pyrazolyle, 1,2,3-triazolyle, 1,2,4-triazolyle, tétrazolyle et 1,2,3-triazinyle.

On peut également citer, à titre d'hétéroaryle, le thiophényle, l'oxazolyle, le furazanyle, le 1,2,4-thiadiazolyle, le naphthyridinyle, le quinoxalinyle, le phtalazinyle, l'imidazo[1,2-a]pyridine, l'imidazo[2,1-b]thiazolyle, le cinnolinyle, le benzofurazanyle, l'azaindolyle, le benzimidazolyle, le benzothiophényle, le thiénopyridyle, le thiénopyrimidinyle, le pyrrolopyridyle, l'imidazopyridyle, le benzoazaindole, le 1,2,4-triazinyle, l'indolizinyle, l'isoxazolyle, l'isoquinolinyle, l'isothiazolyle, le purinyle, le quinazolinyle, le quinolinyle, l'isoquinolyle, le 1,3,4-thiadiazolyle, le thiazolyle, l'isothiazolyle, le carbazolyle, ainsi que les groupes correspondants issus de leur fusion ou de la fusion avec le noyau phényle.

Selon un mode de réalisation, dans les complexes selon l'invention, le lanthanide est choisi dans le groupe constitué de Yb, Nd, Ho, Tm, Sm, Dy, Eu, Pr et Er. La teneur en lanthanide respecte la stœchiométrie d'un cation lanthanide par molécule de TTHA.

La présente demande décrit également un conjugué comprenant une molécule biologique et un complexe tel que défini ci-dessus, dans lequel ledit complexe est lié à la molécule biologique par l'intermédiaire d'un linker, ladite molécule biologique étant choisie dans le groupe constitué des anticorps, des protéines, des peptides, des glucides, des lipides, des polysaccharides, des acides gras, des acides aminés, des acides désoxyribonucléiques, des acides ribonucléiques, des oligonucléotides, des médicaments et des ligands.

La présente invention concerne également l'utilisation d'un complexe tel que défini ci-dessus, comme chromophore fluorescent.

Selon l'invention, le terme "chromophore fluorescent" désigne une molécule pouvant réémettre de la lumière après excitation avec un rendement quantique supérieur à 10⁻⁶ (10⁻⁴%).

Les complexes selon l'invention peuvent notamment être utilisés dans le domaine de l'imagerie cellulaire, de l'imagerie vétérinaire, des analyses sanguines, des biopsies, des analyses de coupes histologiques, des analyses des criblage à haut débit, des essais bioanalytiques sur plaques 96, 396 et 10536 puits ou encore de la chirurgie assistée (guidée) par imagerie.

### DESCRIPTION DES FIGURES

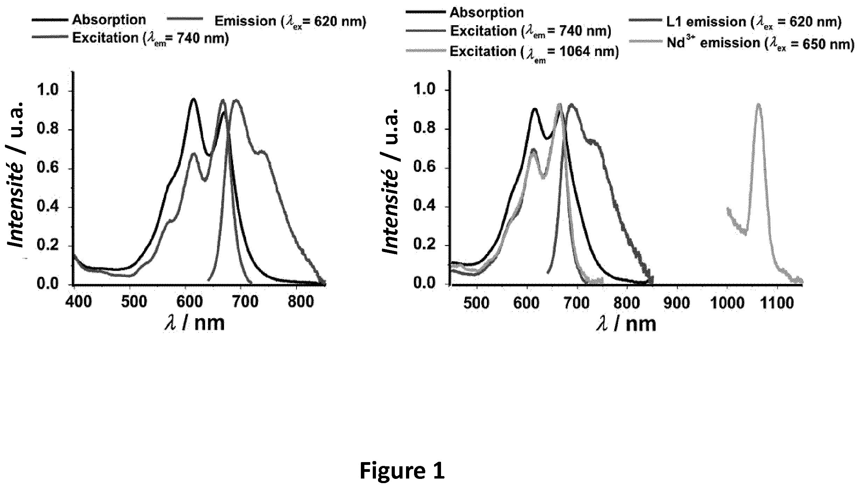

La Figure 1 représente des spectres d'absorption, d'excitation et d'émission normalisés du ligand L1 (à gauche) et du complexe L1Nd³⁺ (à droite, 100 µM, tampon HEPES, pH =7.5, mesurés à température ambiante).

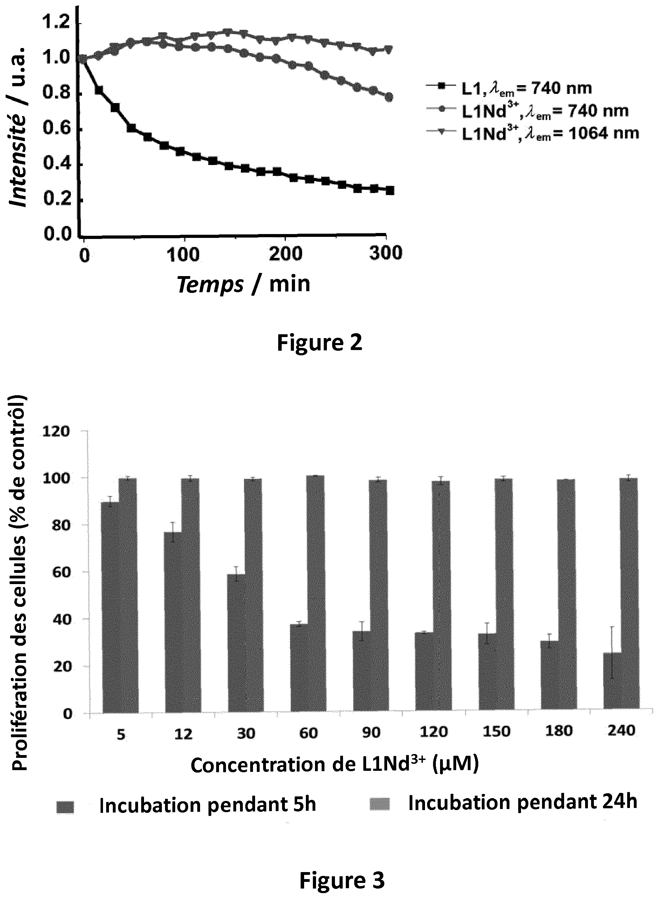

La Figure 2 concerne les résultats des tests de photostabilité obtenus sur des solutions 100 µM solutions de ligand L1 et de complexe L1Nd³⁺ dans le tampon HEPES (pH = 7.5) sous illumination continue à 630 nm à température ambiante.
La Figure 3 concerne des résultats des tests de cytotoxocité obtenus avec l'aide du test « Alamar Blue » effectué sur des cellules HeLa incubées avec des concentrations variables de complexes L1Nd³⁺ durant 5h (colonnes de gauche) et 24h (colonnes de gauche).

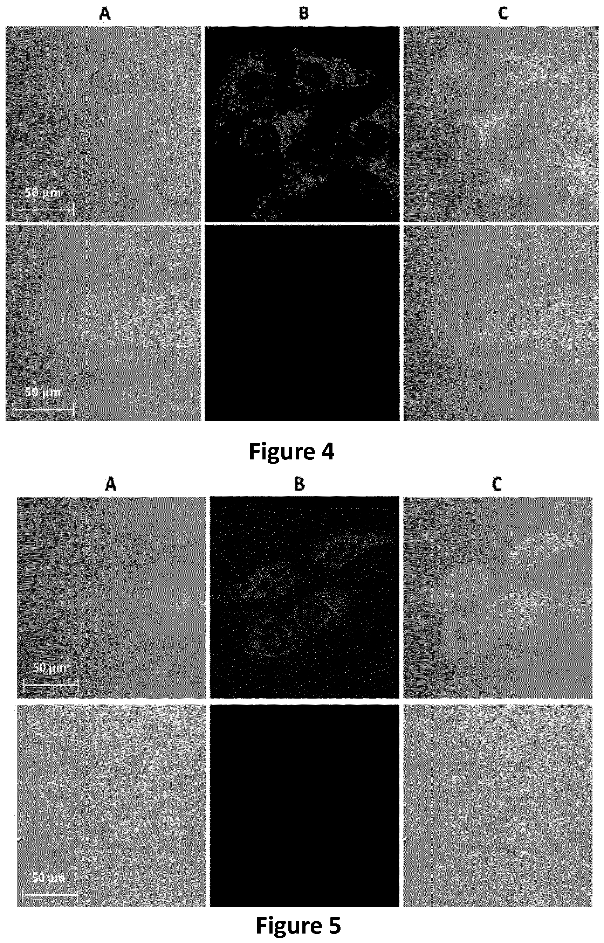

La Figure 4 représente des images obtenues lors d'expériences de microscopie confocale conduites sur des cellules HeLa (images du haut) incubées avec une solution 5 µM du complexe L1Nd³⁺ durant 3 heures et (images du bas) sur des cellules de contrôle qui n'ont pas été incubées avec le complexe L1Nd³⁺. (A) Image de transillumination visible, (B) Emission centrée sur le ligand L1 (*λ*ₑₓ: 633 nm; *λ*ₑₘ : 650-750 nm), (C) Résultat de la fusion des images. Images de plan unique sélectionné au milieu du volume cellulaire (dans le plan du noyau).
La Figure 5 représente des images obtenues des expériences de microscopie confocale conduites sur des cellules HeLa (images du haut) incubées avec une solution 50 µM en L1Nd³⁺ durant 3h et (images du bas) sur des cellules de contrôle qui ont été traitées de manière similaire à l'exception de l'absence d'incubation avec le complexe L₁ Nd³⁺. (A) Transillumination visible, (B) Emission centrée sur le ligand L1 (*λ*ₑₓ : 633 nm; *λ*ₑₘ : 650-750 nm), (C) Résultat de la fusion des images. Images de plan unique localisées au milieu du volume cellulaire (dans le plan du noyau).

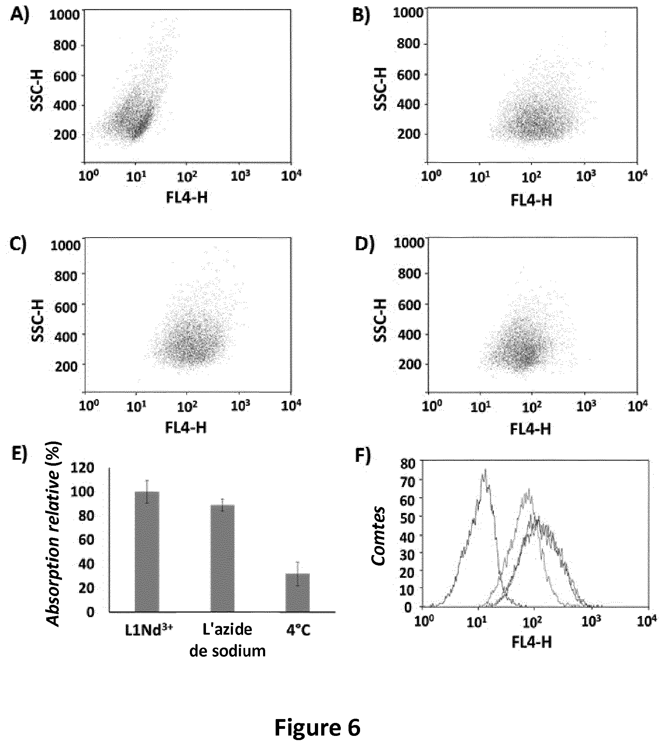

La Figure 6 concerne des résultats des expériences de cytométrie en flux. Les cellules HeLa ont été observées après 3h d'incubation dans le milieu de culture Opti-MEM + 2% FBS : (A) cellules de contrôle qui ont été traitées de manière similaire à l'exception de l'absence d'incubation avec le complexe L1Nd³⁺; (B) une solution 5 µM de complexe L1Nd³⁺; (C) une solution contenant 5 µM de L1Nd³⁺ et 100 mM d'azide de sodium et (D) une solution de 5 µM de L1Nd³⁺ à 4°C. Les conditions de mesure de (C) et (D) impliquent que les cellules ont été soit pré-incubées avec une solution de 100 mM d'azide de sodium ou placées à 4°C durant 30 min avant d'être incubées avec la solution de L1Nd³⁺. Les diagrammes en pointillé représentent les distributions de cellules en mode « 2FSC (forward scatter) » et « SSC (side scatter) » ; (E) Quantification de l'incorporation cellulaire du complexe L1Nd³⁺ mesurée sous différentes conditions expérimentales. Afin de faciliter la lecture des données, l'intensité de fluorescence provenant des cellules HeLa incubées avec le complexe L1Nd³⁺ sans inhibition du transport a été normalisée à la valeur de 100% d'incorporation avec un RSD de ± 9.38 ; (F) Histogrammes correspondant aux conditions expérimentales A, B, C, D.

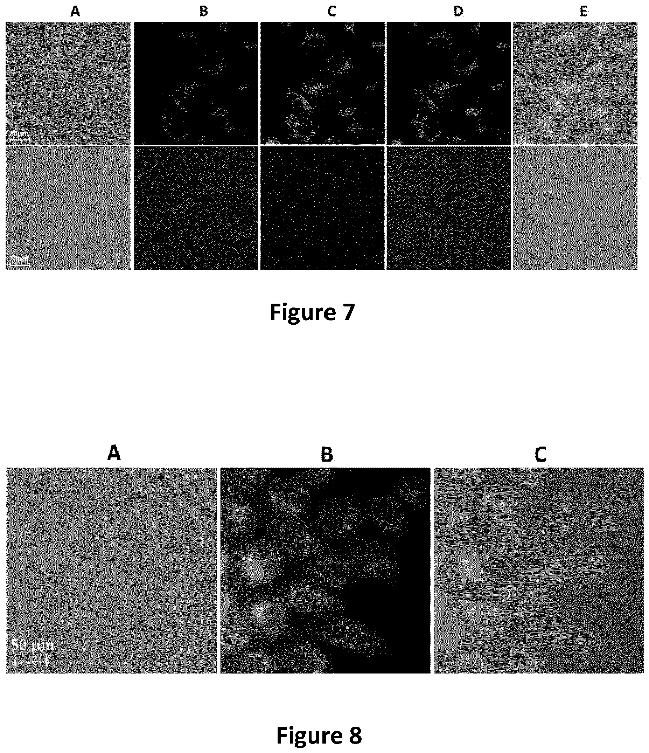

La Figure 7 concerne des résultats des expériences de microscopie d'épifluorescence conduites sur des cellules HeLa. La colocalisation du complexe L1Nd³⁺ dans les cellules HeLa (haut) après 3h d'incubation avec une solution 5 µM de complexe L1Nd³⁺ et 30 min d'incubation avec une solution 50 nM de « LysoTracker Yellow ». (A) Transillumination visible, (B) Emission du "LysoTracker Yellow" (*λ*ₑₓ: filtre à 485 nm de bande passante de 20 nm; *λ*ₑₘ : filtre à 540 nm de bande passante de 50 nm, temps d'exposition de 800 ms), (C) Emission de L1Nd³⁺ dans le proche-infrarouge (*λ*ₑₓ : filtre à 655 nm de bande passante 40 nm; *λ*ₑₘ : filtre coupe bas à 805 nm, temps d'exposition de 20s), (D) Image résultant de la combinaison de l'image de l'émission du « LysoTracker Yellow » et de l'image de l'émission du Nd³⁺, (E) image résultant de la combinaison de l'image de fluorescence du « LysoTracker Yellow », de l'émission centrée sur le Nd³⁺ et de transillumination visible. (Bas) cellules de contrôle qui ont été traitées de manière similaire à l'exception de l'absence d'incubation avec le complexe L1Nd³⁺. Objectif offrant un grossissement de 63x.
La Figure 8 représente des résultats des expériences de microscopie d'épifluorescence conduites sur des cellule HeLa incubées durant 3h avec une solution 50 µM de complexe L1Nd³⁺ (*λ*ₑₓ : 655 nm, bande passante de 40 nm ; filtre coupe bas à 805 nm, 15s d'exposition). (A) Transillumination visible, (B) Emission proche-infrarouge (*λ*ₑₓ : 655 nm, bande passante de 40 nm ; *λ*ₑₘ : 805 nm, filtre coupe bas à 805 nm, 20s de temps d'exposition), (C) Combinaison des images (A) et (B). Objectif de grossissement 63x.

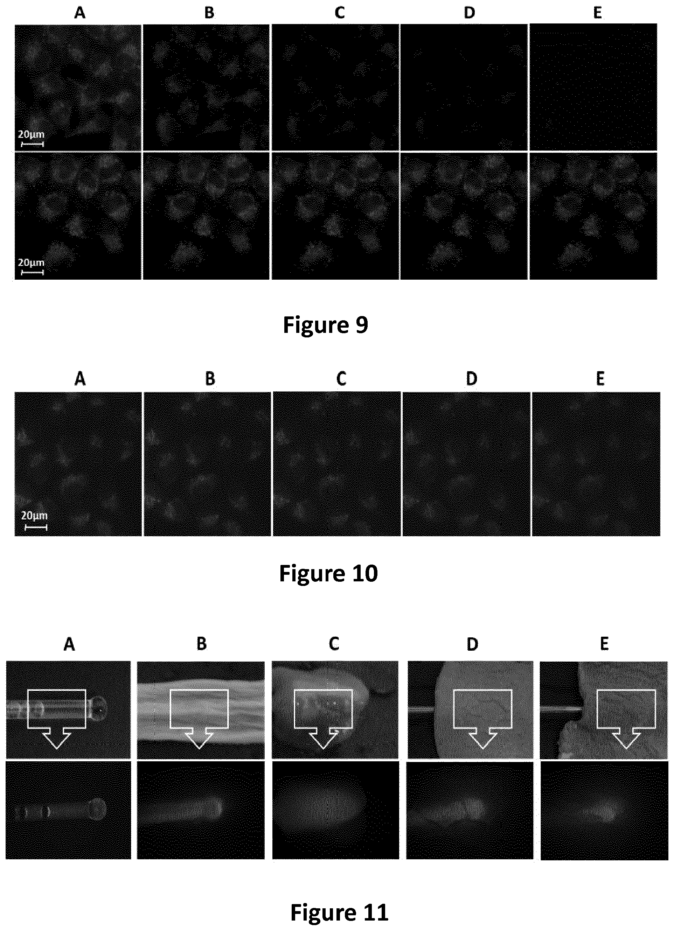

La Figure 9 représente des résultats des tests de photoblanchiment. Des cellules HeLa incubées durant 3h avec une solution 5 µM de complexe L1Nd³⁺ ou 30 min avec une solution 50nM de « LysoTracker Deep Red » après exposition sous excitation continue de lumière sélectionnée par un filtre à 655 nm de bande passante de 40 nm de différentes durées : (A) 0 s, (B) 20 s, (C) 45 s, (D) 70 s, (E) 95 s. (Haut) Emission du « LysoTracker Deep Red » (*λ*ₑₓ : filtre à 655 nm bande passante de 40 nm ; *λ*ₑₘ : filtre à 716 nm de bande passante de 40 nm, temps d'exposition de 300 ms). (Bas) Emission provenant du chromophore anthraquinone localisée sur le complexe L1Nd³⁺ (*λ*ₑₓ : filtre à 655 nm de bande passante de 40 nm ; *λ*ₑₘ ; émission à 716 nm de bande passante de 40 nm, exposition de de 600 ms). Objectif de grossissement 63x.
La Figure 10 représente des résultats des expériences de tests de photoblanchiment. Des cellules HeLa incubées avec une solution 5µM de L1Nd³⁺ durant 3h puis exposées à une illumination continue contrôlée par le biais d'un filtre à 655 nm de bande passante de 40nm de différentes durées : (A) 0 s, (B) 120 s, (C) 245 s, (D) 370 s, (E) 495 s. Détection de l'émission proche-infrarouge (*λ*ₑₓ : filtre à 655 nm de bande passante de 40 nm ; *λ*ₑₘ : filtre coupe bas à 805 nm, temps d'exposition de 20s, objectif de grossissement de 63X).
La Figure 11 représente des résultats des expériences de macroscopie d'épifluorescence proche-infrarouge conduites avec une solution de 200 µM de L1Nd³⁺ placée dans un capillaire en verre (A) et couverte de tissus biologiques de différentes origines et épaisseurs : peau de poulet de 0,6 mm (B), foie de poulet de 2 mm (C), chair de bœuf de 1,6 mm (D), chair de porc de 3 mm (E). (Haut) Transillumination visible et (bas) images de luminescence proche-infrarouge (*λ*ₑₓ: 685 nm (laser pulsé) ; *λ*ₑₘ : filtre passe bas à 785 nm, temps d'exposition de 18 s).

### EXEMPLES

### Exemple 1 : Synthèse de composés (C) selon l'invention

### 1. Préparation d'antennes de type aza-BODIPY

### 1.1. Préparation du tert-butyl N-[2-[[2-[4-[(2Z)-2-[1-difluoroboranyl-3-(4-méthoxyphényl)-5-phényi-pyrrol-2-yl]imino-5-phényl-pyrrol-3-yl]phénoxy] acétyl]amino]éthyl]carbamate (10)

Cette antenne (10) est obtenue selon le schéma réactionnel ci-dessous :

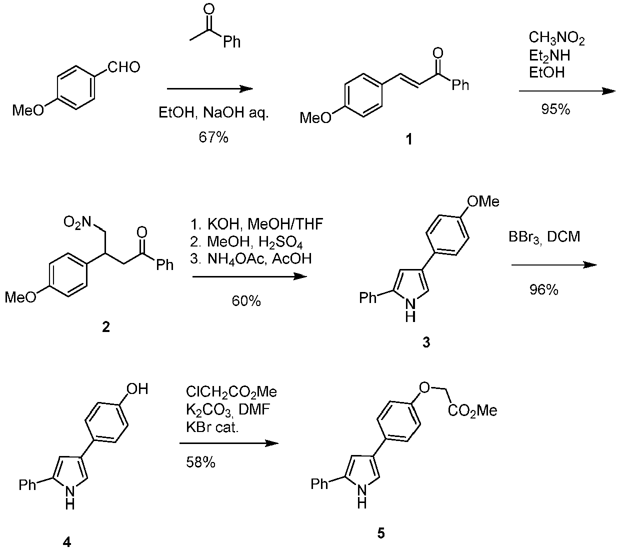

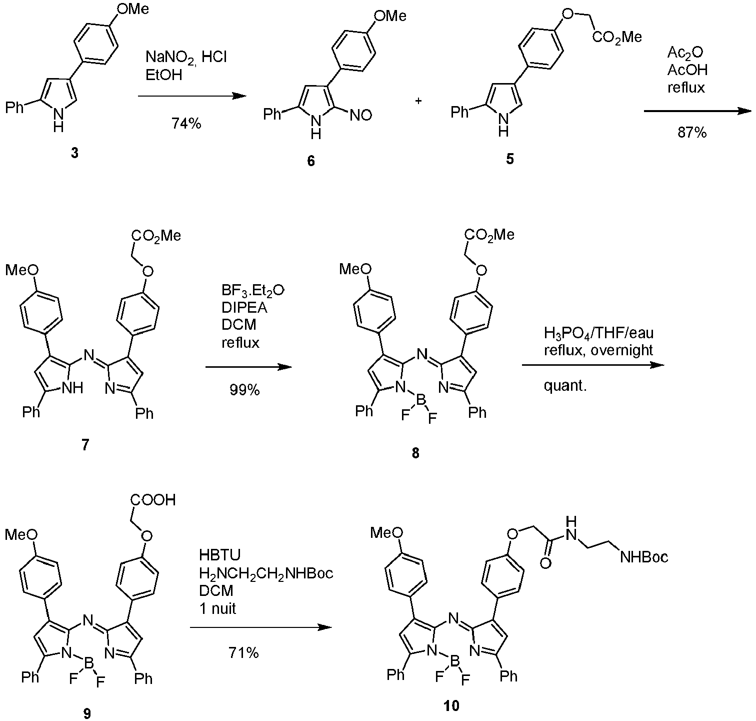

### Synthèse de (E)-3-(4-méthoxyphényl)-1-phénylprop-2-èn-1-one (1)

Dans un ballon sont dissous 8,94 mL de para-anisaldéhyde (Sigma) (10 g, 73,45 mmol, 1 eq.) et 8,58 mL d'acétophénone (Sigma) (8,825 g, 73,45 mmol, 1 eq.) dans 150 mL d'éthanol. Le ballon est plongé dans un bain de glace et 5,876 g de soude (149,9 mmol, 2 eq.) dissous dans 50 mL d'eau sont ajoutés goutte à goutte. On laisse la solution remonter à température ambiante et être agitée toute la nuit. Le lendemain, le ballon est mis dans un bain de glace et de l'eau froide est ajoutée au mélange. Un précipité jaune se forme et le mélange est filtré sur fritté et lavé à l'eau. Le précipité est ensuite recristallisé dans l'éthanol fournissant 11,698 g de la chalcone **1** (49.1 mmol, 67%) sous forme de cristaux blancs.

¹H RMN (250 MHz, Chloroforme-d) δ 8,06 - 7,97 (m, 2H), 7,79 (d, *J* = 15,0 Hz, 1H), 7,64 - 7,59 (m, 2H), 7,58-7,45 (m, 3H), 7,42 (d, *J* = 15,6 Hz, 1H), 6,98-6,91 (m, 2H), 3,86 (s, 3H).

### Synthèse de 3-(4-méthoxyphényl)-4-nitro-1-phénylbutan-1-one (2)

Dans un ballon sont dissous 0,546 g (2,29 mmol, 1 eq.) de **1,** 0,621 mL de nitrométhane (Sigma) (11,46 mmol, 5 eq.) et 1,185 mL de diéthylamine (Sigma) (11,46 mmol, 5 eq.) dans 150 mL de méthanol. La solution est chauffée à reflux toute la nuit. Une fois la réaction terminée, le solvant est évaporé sous vide et le résidu dissous dans le dichlorométhane. La solution est lavée avec une solution de KHSO₄ 1M puis NaCl saturée. Les phases organiques sont rassemblées, séchées sur MgSO₄, filtrée et concentrées sous vide. Le résidu est purifié par chromatographie sur silice avec éther de pétrole/acétate d'éthyle fournissant la nitrobutanone **2** (0,654 g, 2,18 mmol, 95%) sous forme d'un huile visqueuse jaune pâle.

¹H RMN (250 MHz, Chloroforme-d) δ 7,96 - 7,88 (m, 2H), 7,63 - 7,53 (m, 1H), 7,51 - 7,41 (m, 2H), 7,25 - 7,16 (m, 2H), 6,91 - 6,81 (m, 2H), 4,80 (ddd, *J* = 12,3, 6,7, 0,5 Hz, 1H), 4,65 (ddd, *J* = 12,3, 7,9, 0,4 Hz, 1H), 4,18 (p, *J* = 7,0 Hz, 1H), 3,78 (s, 3H), 3,42 (dd, *J* = 7,0, 2,1 Hz, 2H).

### Synthèse de 4-(4-méthoxyphényl)-2-phényl-1H-pyrrole (3)

Dans un ballon sont dissous 3,5 g de nitrobutanone **2** (11.69 mmol, 1 eq.) et 5 eq. de KOH dans 100 mL d'un mélange MeOH/THF (1 :2). La solution est agitée à température ambiante durant une heure et est ensuite ajoutée goutte à goutte à une solution de H₂SO₄ concentré (2 mL/mmol) dissoute dans 100 mL de MeOH à 0°C. Une fois l'addition terminée, le bain de glace est enlevé et la solution agitée à température ambiante pendant 1 h. Le mélange est ensuite versé dans un erlenmeyer contenant de l'eau et de la glace et la solution est neutralisée en ajoutant une solution de soude 4M. Une fois neutralisée, le mélange est extrait avec du dichlorométhane et la phase organique est séchée sur MgSO₄, filtrée et le solvant évaporé sous pression réduite. Le résidu est dissous dans 100 mL d'acide acétique glacial et 5 eq. d'acétate d'ammonium (Sigma) sont ajoutés. La solution est chauffée à reflux durant une heure pendant laquelle la couleur de la solution évolue du jaune au bleu profond. La solution est ramenée à température ambiante et l'acide acétique évaporé sous pression réduite. Le solide noir est alors dissous dans le dichlorométhane et la solution est lavée plusieurs fois avec une solution saturée de bicarbonate de sodium et de saumure. La phase organique est séchée sur MgSO₄, filtrée et concentrée sous pression réduite. Le résidu est ensuite dissous dans un minimum de dichlorométhane et de l'éther de pétrole est ajouté lentement jusqu'à ce que se forme un précipité. Le précipité est filtré sur Büchner et lavé plusieurs fois à l'éther de pétrole. Le pyrrole **3** est obtenu avec un rendement de 60% (1.742 g, 6.99 mmol) sous forme d'une poudre légèrement colorée.

¹H RMN (250 MHz, DMSO-*d*₆) δ 11,32 (s, 1H), 7,66 (dt, *J* = 7,7, 1,1 Hz, 2H), 7,57 - 7,48 (m, 2H), 7,36 (dd, *J* = 8,4, 7,0 Hz, 2H), 7,21 (dd, *J* = 2,8, 1,7 Hz, 1H), 7,20 - 7,12 (m, 1H), 6,94 - 6,84 (m, 3H), 3,75 (s, 3H).

### Synthèse de 4-(5-phényl-1H-pyrrol-3-yl)phénol (4)

Dans un ballon sous argon sont dissous 0,546 g de pyrrole **3** (2,19 mmol, 1 eq.) dans 100 mL de dichlorométhane anhydre. La solution est refroidie à -78°C et 5,48 mL d'une solution de BBr₃ 1M dans le dichlorométhane (Sigma) (5,48 mmol, 2,5 eq.) sont ajoutés lentement. La réaction est agitée 3 h à -78°C puis toute la nuit à température ambiante. Le lendemain, la solution est refroidie à -78°C et du MeOH est ajouté au mélange. La solution est agitée pendant une heure puis elle est diluée dans du dichlorométhane et lavée avec du NaCl saturé. La phase organique est séchée sur MgSO₄, filtrée et concentrée sous pression réduite. Le résidu est dissous dans un minimum de dichlorométhane et le produit est précipité par ajout lent d'éther de pétrole. Le précipité est filtré sur Büchner et lavé à l'éther de pétrole fournissant 0,495 g (2,10 mmol, 96%) du pyrrole **4** sous forme d'une poudre blanche légèrement violacée.

¹H RMN (250 MHz, DMSO-*d*₆) δ 11.25 (s, 1H), 9.15 (s, 1H), 7.69 - 7.61 (m, 2H), 7.43 - 7.31 (m, 4H), 7.20 - 7.11 (m, 2H), 6.81 (dd, *J* = 2.7, 1.7 Hz, 1H), 6.77 - 6.69 (m, 2H).

¹H RMN (250 MHz, acétone-de) δ 10,48 (s, 1H), 8,09 (s, 1H), 7,71 - 7,65 (m, 2H), 7,49 - 7,41 (m, 2H), 7,41 - 7,31 (m, 2H), 7,21 - 7,14 (m, 2H), 6,85 (dd, *J* = 2,8, 1,7 Hz, 1H), 6,84 - 6,80 (m, 2H).

¹³C RMN (63 MHz, acétone) δ 156,27, 134,16, 133,40, 129,58, 128,76, 126,92, 126,80, 126,55, 124,38, 116,24, 115,94, 104,14.

LRMS : calculé : 235,0997, mesuré [M+H]⁺ : 236,1

IR (cm⁻¹) : 3443, 3300, 1600, 1581, 1494, 1244, 1132, 921, 834, 804, 778, 751, 717, 690, 609.

Mp : 209°C

### Synthèse de méthyl 2-(4-(5-phényl-1H-pyrrol-3-yl)phénoxy)acétate (5)

Dans un ballon sont dissous 0,725 g de **4** (3,08 mmol, 1 eq.) dans 60 mL de DMF. 1,278 g de K₂CO₃ (9,24 mmol, 3 eq.), 1,08 mL de chloroacétate de méthyle (Sigma) (12,33 mmol, 4 eq.) et une quantité catalytique de bromure de potassium sont ajoutés à la solution qui est agitée à température ambiante toute la nuit. La solution est ensuite extraite à l'éther diéthylique et lavée trois fois avec de la saumure. La phase aqueuse est ensuite extraite trois fois avec de l'éther diéthylique. Les phases organiques sont rassemblées et séchées sur MgSO₄, filtrées et le solvant évaporé sous vide. Le résidu est dissous dans un minimum de dichlorométhane et de l'éther de pétrole est ajouté jusqu'à formation d'un précipité. Le précipité est filtré sur Büchner et lavé à l'éther de pétrole pour obtenir 0,553 g de **5** sous forme d'une poudre blanc cassé.

¹H RMN (250 MHz, Chloroforme-d) δ 8,43 (s, 1H), 7,55 - 7,46 (m, 4H), 7,44 - 7,35 (m, 2H), 7,25 - 7,19 (m, 1H), 7,07 (dd, *J* = 2,7, 1,7 Hz, 1H), 6,97 - 6,88 (m, 2H), 6,76 (dd, *J* = 2,8, 1,7 Hz, 1H), 4,66 (s, 2H), 3,82 (s, 3H).

¹³C RMN (63 MHz, CDCl₃) δ 169,57, 156,10, 133,02, 132,51, 129,56, 128,93, 126,45, 126,38, 126,11, 123,83, 114,98, 114,94, 103,86, 65,58, 52,25.

LRMS : calculé : 307,1208, mesuré [M+H]⁺ : 308,4

IR (cm⁻¹) : 3430, 2941, 1759, 1600, 1581, 1496, 1438, 1212, 1178, 1131, 1075, 834, 801, 774, 758, 719, 693.

Mp : 165°C.

### Synthèse de 3-(4-méthoxyphényl)-2-nitroso-5-phényl-1H-pyrrole (6)

Dans un ballon à température ambiante sont dissous 0,593 g de pyrrole **3** (2,38 mmol, 1 eq.) dans 50 mL d'éthanol et 0,48 mL d'HCI concentré (0,2 mL/mmol). 0,189 g de nitrite de sodium (2,74 mmol, 1,15 eq.) dissous dans de l'eau (concentration 0,6 mol/L) sont ajoutés goutte à goutte. La solution est agitée 30 minutes et est ensuite refroidie à 0°C. Une deuxième portion d'HCI concentré (2,38 mL, 1 mL/mmol) est ajoutée. La solution est agitée une heure puis, elle est dissoute dans du dichlorométhane et lavée avec de la saumure. La phase organique est séchée, filtrée et concentrée sous pression réduite. Le résidu est dissous dans un volume minimal d'éthanol et un excès de solution aqueuse d'acétate de sodium et de glace sont ajoutés et le mélange est agité une heure. La solution est ensuite extraite au dichlorométhane et lavée avec de la saumure. La phase organique est séchée sur MgSO₄, filtrée et concentrée sous vide. Le résidu est ensuite dissous dans un volume minimal de dichlorométhane et le produit est précipité par addition lente d'éther de pétrole. Le solide est filtré sur Büchner et lavé avec de l'éther de pétrole. 0,503 g de nitrosopyrrole **6** (1,81 mmol, 76%) sont obtenus sous forme d'une poudre verte.

¹H RMN (250 MHz, Chloroforme-d) δ 8,20 - 8,13 (m, 2H), 7,79 (dd, *J* = 6,9, 3,0 Hz, 2H), 7,51 (dd, *J* = 5,1, 1,8 Hz, 3H), 7,07 (s, 1H), 7,02 (d, *J* = 8,9 Hz, 2H), 3,89 (s, 3H).

### Synthèse de (Z)-méthyl 2-(4-(2-((3-(4-méthoxyphényl)-5-phényl-1H-pyrrol-2-yl)imino)-5-phényl-2H-pyrrol-3-yl)phénoxy)acétate (7)

Dans un ballon sont dissous dans 20 mL d'acide acétique glacial 0,112 g de nitrosopyrrole **6** (0,40 mmol, 1 eq.), 0,124 g de pyrrole **5** (0,40 mmol, 1 eq. et 0,40 mL d'anhydride acétique. La solution est agitée et chauffée à reflux pendant une heure durant laquelle la coloration passe au bleu foncé. La solution est ensuite refroidie et le solvant évaporé sous pression réduite. Le résidu est ensuite dissous dans du dichlorométhane et la solution est lavée avec NaHCO₃ saturé et NaCl saturé. La phase organique est séchée sur MgSO₄, filtrée et concentrée sous vide. Le résidu est ensuite dissous dans un minimum de dichlorométhane et de l'éther de pétrole est ajouté jusqu'à formation d'un précipité qui est filtré sur Büchner et lavé à l'éther de pétrole. L'azadipyrrométhène **7** est obtenu sous forme d'une poudre bleu foncé (0,197 g, 0,35 mmol, 87%).

¹H RMN (250 MHz, Chloroform-*d*) δ 8,05 - 7,98 (m, 4H), 7,93 (ddd, *J* = 7,8, 6,4, 1,5 Hz, 4H), 7,58 - 7,44 (m, 7H), 7,11 (s, 1H), 7,09 (s, 1H), 6,96 (dd, *J* = 8,9, 1,8 Hz, 4H), 4,71 (s, 2H), 3,90 (s, 3H), 3,85 (s, 3H).

IR (cm⁻¹) : 3426, 2948, 1758, 1597, 1495, 1211, 1175, 1075, 1002, 902, 833, 801, 772, 759, 745, 718, 692, 637, 605.

Mp : 230°C.

HRMS (ESI) : m/z calculé pour [C₃₆H₃₀N₃O₄] : 568,223083, mesuré 568,222834 (-0,4 ppm)

### Synthèse de (Z)-méthyl 2-(4-(2-((1-(difluoroboryl)-3-(4-méthoxyphényl)-5-phényl-1H-pyrrol-2-yl)imino)-5-phényl-2H-pyrrol-3-yl)phénoxy)acétate (8)

Dans un ballon sous argon sont dissous 0,183 g d'azadipyrrométhène **7** (0,32 mmol, 1 eq.) et 0,548 mL de DIPEA (Sigma) (3,22 mmol, 10 eq.) dans du dichlorométhane fraîchement distillé. Après quelques minutes d'agitation à température ambiante, 0,613 mL de BF₃.Et₂O (Sigma) distillé (4,84 mmol, 15 eq.) sont ajoutés et la solution est portée à reflux durant deux heures. La solution est ensuite refroidie à température ambiante et la phase organique est lavée avec de la saumure trois fois. La phase aqueuse est ensuite réextraite trois fois avec du dichlorométhane et les phases organiques combinées sont séchées sur MgSO₄, filtrées et concentrées sous vide. Le résidu est purifié par chromatographie sur silice avec un gradient de PE/DCM. L'azabodipy **8** est obtenu sous forme d'une poudre bleu foncé iridescente (0,196 g, 0,318 mmol, 99%).

¹H RMN (400 MHz, Acétone-de) δ 8,23 (dd, *J* = 8,8, 6,2 Hz, 4H), 8,18 - 8,09 (m, 4H), 7,58 - 7,46 (m, 6H), 7,33 (d, *J* = 1,4 Hz, 2H), 7,14 (dd, *J* = 9,0, 7,2 Hz, 4H), 4,88 (s, 2H), 3,92 (s, 3H), 3,79 (s, 3H).

¹³C RMN (101 MHz, Acétone) δ 169,66, 162,39, 160,53, 132,72, 131,94, 131,87, 131,70, 131,62, 130,55, 130,51, 129,36, 125,87, 118,92, 115,83, 115,26, 65,70, 55,88, 52,29.

¹⁹F RMN (235 MHz, Acétone-de) δ -130,38 (dd, *J* = 62,8 - 31,4 Hz).

HRMS (ESI): m/z calculé pour [C₃₅H₂₃BF₂N₃O₄]: 616,221996, mesuré 616,221217 (-1,3 ppm)

Mp : 179°C

IR (cm⁻¹) : 3288, 2918, 2584, 1758, 1728, 1601, 1504, 1487, 1454, 1388, 1277, 1252, 1228, 1175, 1129, 1100, 1068, 1024, 999, 970, 929, 904, 868, 836, 818, 767, 742, 690, 641, 615.

### Synthèse de l'acide (Z)-2-(4-(2-((1-(difluoroboryl)-3-(4-méthoxyphényl)-5-phényl-1H-pyrrol-2-yl)imino)-5-phényl-2H-pyrrol-3-yl)phénoxy)acétique (9)

Dans un ballon est dissous 0,308 g d'azabodipy **8** (0,5 mmol, 1 eq.) dans un mélange THF/eau/H₃PO₄ (50 mL : 25 mL : 10 mL). La solution est agitée sous reflux 20h jusqu'à ce qu'aucune trace de l'ester ne soit visible par CCM. Après refroidissement, la solution est extraite au dichlorométhane. La phase organique est lavée avec de la saumure puis, les phases aqueuses sont réextraites au dichlorométhane jusqu'à ce qu'aucune coloration bleue ne soit observée dans la phase aqueuse. La phase organique est séchée sur MgSO₄, filtrée et concentrée sous pression réduite. On peut éventuellement purifier le résidu par chromatographie avec DCM/MeOH si de l'ester est encore présent dans le brut. L'azabodipy **9** est obtenu sous forme d'un solide bleu foncé (0,294 g, 0,49 mmol, 98%).

¹H RMN (250 MHz, Acétone-de) δ 8,27-8,18 (m, 4H), 8,18-8,09 (m, 4H), 7,57-7,48 (m, 6H), 7,32 (d, *J* = 1,8 Hz, 2H), 7,19-7,08 (m, 4H), 4,86 (s, 2H), 3,91 (s, 3H).

HRMS (ESI) : m/z calculé pour [C₃₅H₂₇BF₂N₃O₄] : 602,206329, mesuré 602,205753 (1,0 ppm)

### Synthèse de tert-butyl N-[2-[[2-[4-[(2Z)-2-[1-difluoroboranyl-3-(4-méthoxyphényl)-5-phényl-pyrrol-2-yl]imino-5-phényl-pyrrol-3-yl]phénoxy] acétyl]amino]éthyl]carbamate (10)

Dans un ballon sous argon sont dissous 0,080 g de **9** (0,13 mmol, 1 eq.), 0,070 mL de DIPEA (Sigma) (0,40 mmol, 3 eq.) et 0,076 g de HBTU (Sigma) (0,20 mmol, 1,5 eq.) dans 5 mL de dichlorométhane et 1 mL d'acétonitrile distillés. La réaction est agitée 15 minutes puis on ajoute 0,032 g de Boc-éthylène diamine (0.20 mmol, 1.5 eq.) dissous dans 2 mL de dichlorométhane anhydre. La réaction est agitée 1h30. Ensuite, la solution est extraite au dichlorométhane et lavée successivement avec KHSO₄ 1M, NaHCO₃ sat. et NaCl sat. Les phases organiques sont rassemblées et séchées sur MgSO₄, filtrées et concentrées sous vide. Le résidu est ensuite purifié par chromatographie sur silice avec un mélange PE/EA fournissant 0,069 g d'un solide bleu profond aux reflets métalliques (0,093 mmol, 71%).

¹H RMN (250 MHz, Chloroforme-d) δ 8,10 - 7,98 (m, 8H), 7,51 - 7,44 (m, 6H), 7,02 (dd, *J* = 9,0, 8,1 Hz, 4H), 6,94 (d, *J* = 1,1 Hz, 2H), 4,88 (s, 1H), 4,58 (s, 2H), 3,92 (s, 3H), 3,50 (q, *J* = 5,6 Hz, 2H), 3,35 (dd, *J* = 12,1, 6,0 Hz, 2H), 2,80 (s, 3H), 1,43 (s, 9H).

¹³C RMN (101 MHz, CDCl₃) δ 131,13, 131,08, 130,96, 130,79, 129,66, 128,69, 125,36, 117,98, 117,94, 115,03, 114,42, 67,41, 55,65, 38,76, 28,49.

HRMS : [M+H]⁺ C₂₄H₄₁BF₂N₅O₅ m/z calculé 744,317054, mesuré 744316900 (0.2 ppm).

### 1.2. Préparation du 2-[4-[(2Z)-2-[5-[4-[4-(tert-butoxycarbonylamino) butoxy]phényl]-1-difluoroboranyl-3-phényl-pyrrol-2-yl]imino-5-phényl-pyrrol-3-yl]phénoxy]acétate de méthyle (19)

Cette antenne (19) est obtenue selon le schéma réactionnel ci-dessous :

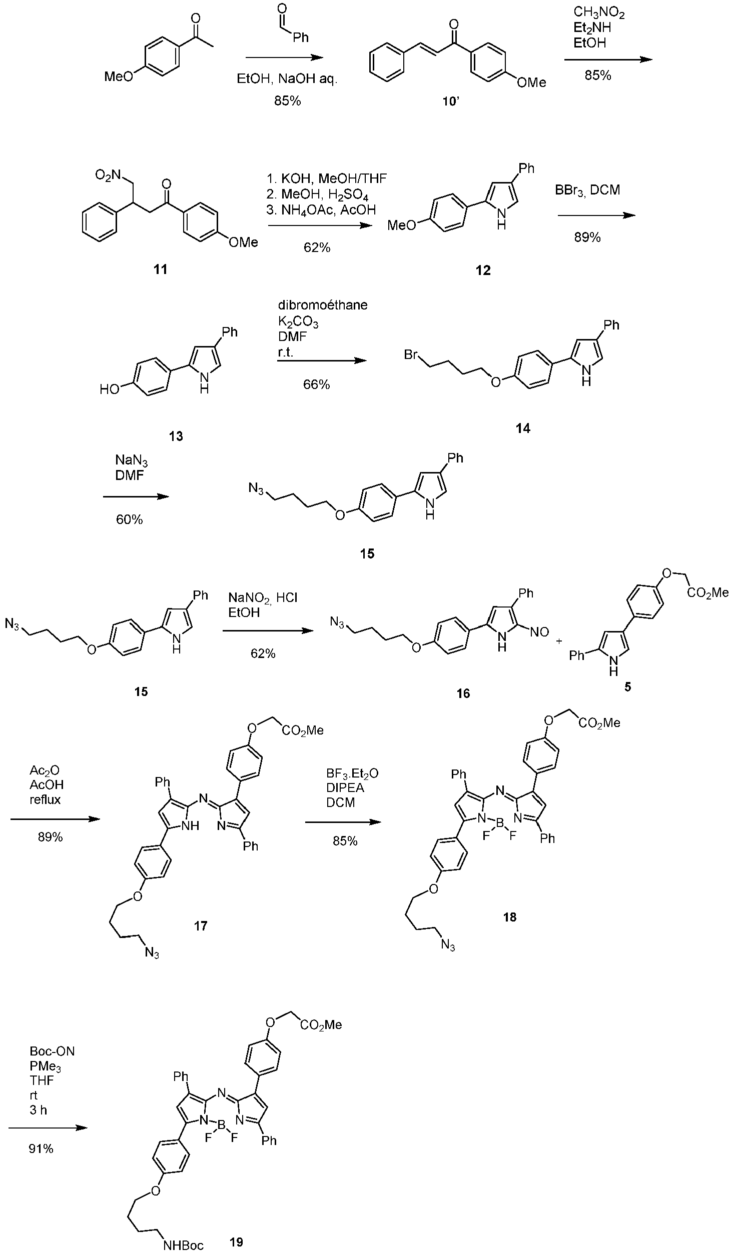

### Synthèse de (E)-1-(4-méthoxyphényl)-3-phényl-prop-2-èn-1-one (10')

Dans un ballon sont dissous 15 g de paraméthoxyacétophénone (Sigma) (0,1 mol, 1 eq.), 10,1 mL de benzaldéhyde (Sigma) (0,1 mol, 1 eq.) et 400 mg de NaOH (10 mmol, 0,1 eq.) dans du méthanol. La solution est agitée à reflux toute la nuit. De l'eau froide est ensuite ajoutée au mélange et le précipité formé est filtré et lavé à l'eau. La chalcone **10'** est recristallisée dans le méthanol sous forme de cristaux blancs avec un rendement de 85% (20,254 g, 85 mmol).

RMN ¹H (CDCl₃, 250 MHz): 8,08-8,01 (tt, 2H), 7,80 (d, *J* = 15,7 Hz, 1H), 7,67-7,61 (m, 2H), 7,54 (d, *J* = 15,7 Hz, 1H), 7,44-7,37 (m, 3H), 7,01-6,94 (tt, 2H), 3,87 (s, 3H).

### Synthèse de 1((4-méthoxyphényl)-4-nitro-3-phényl-butan-1-one (11)

Ce composé est synthétisé en appliquant la procédure décrite pour la préparation du composé **2.**

Masse obtenue : 3,205 g ; 10,71 mmol

Rendement 85%.

RMN ¹H (CDCl₃, 400 MHz) : 7,88-7,86 (d, 2H), 7,31-7,21 (m, 5H), 6,90-6,88 (d, 2H), 4,80 (ddd, *J* = 12,6 Hz, 6,4 Hz, 1,3 Hz, 1H), 4,65 (ddd, *J* = 12,6 Hz, 8,3 Hz 1,3 Hz), 4,18 (p, *J* = 7,1 Hz, 1H), 3,82 (s, 3H), 3,42-3,29 (m, 2H).

RMN ¹³C (CDCl₃, 101 MHz) : 195,39, 163,88, 139,39, 130,39, 129,52, 129,06, 127,83, 127,51, 113,93, 79,68, 55,56, 41,22, 39,49.

### Synthèse de 2-(4-méthoxyphényl)-4-phényl-1H-pyrrole (12)

Ce composé est synthétisé en appliquant la procédure décrite pour la préparation du composé **3.**

Masse obtenue : 1,917 g ; 7,69 mmol

Rendement : 62%

RMN ¹H (CDCl₃, 250 MHz): 8,37 (s, 1H), 7,57 (dd, *J* = 8,4 Hz, 1,3 Hz, 2H), 7,48-7,42 (m, 2H), 7,36 (t, *J* = 7,5 Hz, 3H), 7,23-7,16 (m, 1H), 7,11 (dd, *J* = 2,6 Hz, 1,7 Hz, 1H), 6,98-6,91 (m, 2H), 6,71 (dd, *J* = 2,7 Hz, 1,7 Hz, 1H), 3,84 (s, 3H).

RMN ¹³C (CDCl₃, 63 MHz) : 158,63, 135,79, 133,27, 128,77, 126,64, 125,79, 125,73, 125,46, 125,31, 115,00, 114,55, 103,14, 55,51.

### Synthèse de 4-(4-phényl-1H-pyrrol-2-yl)phénol (13)

Ce composé est synthétisé en appliquant la procédure décrite pour la préparation du composé **4.**

Masse obtenue : 180 mg, 0,77 mmol

Rendement : 89%

Mp: > 300°C

HRMS : [M+H]⁺: 236,1070.

IR (cm⁻¹) : u = 3215, 1495, 1249, 1171, 1101, 832, 763, 696

RMN ¹H (Acétone-d6, 250 MHz) : 10,41 (s, 1H), 8,29 (s, 1H), 7,62-7,58 (m, 2H), 7,55-7,51 (m, 2H), 7,33-7,27 (m, 2H), 7,22 (dd, *J* = 2,8 Hz, 1,7 Hz, 1H), 7,15-7,08 (m, 1H), 6,89-6,85 (m, 2H), 6,77 (dd, *J* = 2,8 Hz, 1,8 Hz, 1H).

### Synthèse de 2-[4-(4-bromobutoxy)phényl]-4-phényl-1H-pyrrole (14)

Dans un ballon à température ambiante sont dissous 0,450 g de **13** (1,91 mmol, 1 eq.), 0,794 g de K₂CO₃ (5,74 mmol, 3 eq.) et 0,69 mL de 1,4-dibromobutane (5,74 mmol, 3 eq.) dans du DMF. La réaction est agitée vigoureusement à température ambiante une nuit. Le lendemain, de l'eau est ajoutée à la réaction et le mélange est extrait à l'éther diéthylique, la phase organique est lavée 3 fois avec NaCl sat puis les phases aqueuses sont réextraites avec de l'éther diéthylique. Les phases organiques sont rassemblées, séchées sur MgSO₄, filtrées et concentrées sous vide. Le résidu est dissous dans un minimum de dichlorométhane, et le produit est précipité par ajout d'éther de pétrole, filtré sur Büchner et lavé avec de l'éther de pétrole fournissant 0,466 g de **14** (1,26 mmol, 66%) sous forme d'une poudre blanche.

Mp : 160-162°C

HRMS : [M(⁷⁹Br)+H]⁺: 371,1813, [M(⁸¹Br)+H]⁺ : 373,0814, [M+H]⁺ : 372,0781. IR (cm⁻¹) : u = 3395, 1497, 1247, 830, 802, 751, 692.

RMN ¹H (DMSO-d6, 250 MHz) : 11,28 (s, 1H,), 7,61-7,57 (m, 4H), 7,34-7,31 (d, 2H), 7,28-7,25 (dd, 1H), 7,14-7,07 (tt, 1H), 6,95 (d, *J* = 2,8 Hz, 2H), 6,80 (dd, *J* = 2,7 Hz, 1,7 Hz, 1H), 4,02 (t, *J* = 6,1 Hz, 2H), 3,62 (t, *J* = 6,5 Hz, 2H), 2,01-1,94 (m, 2H), 1,88-1,81 (m, 2H).

RMN ¹³C (DMSO-d6, 63 MHz): 156,82, 135,86, 132,27, 128,48, 125,59, 124,88, 124,76, 124,49, 124,33, 115,71, 114,71, 101,94, 66,59, 34,86, 29,11, 27,42.

### Synthèse de 2-[4-(4-azidobutoxy)phényl]-4-phényl-1H-pyrrole (15)

Dans un ballon sont dissous 0,450 g de **14** (1,22 mmol, 1 eq.) et 0,395 g d'azoture de sodium (Sigma) (6,08 mmol, 5 eq.) dans du DMF. La réaction est agitée à température ambiante une nuit. Le mélange est dissous dans du dichlorométhane est lavé plusieurs fois avec NaCl sat. La phase organique est séchée sur MgSO₄, filtrée et concentrée sous vide. Après dilution dans un minimum de dichlorométhane, le produit **15** est précipité par addition d'éther de pétrole. Le solide est filtré sur Büchner et on obtient 243 mg de **15** (0,73 mmol, 60%) sous forme d'une poudre blanche.

Mp : 177-180°C

HRMS : [M+H]⁺: 333,1707.

IR (cm⁻¹) : u = 3394, 2948, 2875, 2080, 1496, 1246, 831, 752, 692.

RMN ¹H (CDCl₃, 250 MHz) : 8.38 (s, 1H), 7,59-7,55 (m, 2H), 7,45-7,39 (m, 2H), 7,37-7,33 (m, 2H), 7,23-7,17 (m, 1H), 7,10-7,08 (m, 1H), 6,94-6,88 (m, 2H), 6,72 (dd, *J* = 2,6 Hz, 1,5 Hz, 1H), 4,01 (t, *J* = 5,8 Hz, 2H), 3,38 (t, , *J* = 6,3 Hz, 2H), 1,89-1,80 (m, 4H)

RMN ¹³C (CDCl₃, 63 MHz) : 157,86, 135,76, 133,21, 128,77, 126,57, 125,89 , 125,77, 125,41, 125,27, 116,09, 115,09 , 103,09, 67,41, 51,34, 26,66, 25,90.

### Synthèse de 5-[4-(4-azidobutoxy)phényl]-2-nitroso-3-phényl-1H-pyrrole (16)

Ce composé (poudre ocre-dorée) est synthétisé en appliquant la procédure décrite pour la préparation du composé **6.**

Masse obtenue : 67 mg, 0,186 mmol

Rendement : 62%

Point de fusion : 125-126°C

HRMS : [M+H]⁺ : 362,1612.

IR (cm⁻¹) : u = 3280, 2918, 2850, 2092, 1603, 1360, 1258, 1164, 1038, 828, 768, 695, 668.

RMN ¹H (CDCl₃, 250 MHz) : 8,14-8,11 (m, 2H), 7,84 (d, *J* = 8,4 Hz, 2H), 7,46 (dd, *J* = 5,2 Hz, 1,9 Hz, 3H), 7,15 (s, 1H), 6,99 (d, *J* = 8,2 Hz, 2H), 4,06 (t, *J* = 5,8 Hz, 2H), 3,39 (t, *J* = 6,4 Hz, 2H), 1,93-1,78 (m, 4H).

RMN ¹³C (CDCl₃, 63 MHz): 163,72, 162,05, 142,84, 131,91, 129,71, 129,57, 129,29, 128,93, 122,19, 115,47, 115,03, 67,67, 51,27, 26,53, 25,82.

### Synthèse de 2-[4-[(2Z)-2-[[5-[4-(4-azidobutoxy)phényl]-3-phényl-1H-pyrrol-2-yl]imino]-5-phényl-pyrrol-3-yl]phénoxy]acétate de méthyle (17)

Ce composé (poudre bleue intense) est synthétisé en appliquant la procédure décrite pour la préparation du composé **7.**

Masse obtenue : 0,080 g, 0,123 mmol

Rendement : 89%.

Mp : 150-152°C

HRMS : [M+H]⁺ : 651,2717.

IR (cm⁻¹) : u = 2094, 1759, 1600, 1496, 1241, 1166, 903, 806, 764, 694, 675.

RMN ¹H (CDCl₃, 400 MHz) : 8,00 (d, *J* = 7,8 Hz, 4H), 7,92 (d, *J* = 8,3 Hz, 2H), 7,80 (d, *J* = 7,6 Hz, 2H), 7,48 (t, *J* = 7,5 Hz, 2H), 7,39 (dd, *J* = 12,8 Hz, 6,9 Hz, 4H), 7,17 (s, 1H), 6,99 (d, 3H), 6,93 (d, *J* = 8,4 Hz, 2H), 4,69 (s, 2H), 4,06 (t, *J* = 6,1 Hz, 2H), 3,85 (s, 3H), 3,40 (t, *J* = 6,6 Hz, 2H), 1,91 (dt, *J* = 11,4 Hz, 6,1 Hz, 2H), 1,82 (p, *J* = 6,8 Hz, 2H).

RMN ¹³C (CDCl₃, 101 MHz) : 169,49 , 161,27 , 160,76 , 157,61, 153,83, 148,27, 145,30, 145,27, 138,66, 133,76, 131,94, 130,36, 129,28, 129,22, 129,00, 128,32, 128,22, 128,15, 125,91, 125,50, 117,43, 115,16, 114,52, 111,16, 67,56, 65,53, 52,45, 51,32, 26,61, 25,87.

### Synthèse de 2-[4-[(2Z)-2-[5-[4-(4-azidobutoxy)phényl]-1-difluoroboranyl-3-phényl-pyrrol-2-yl]imino-5-phényl-pyrrol-3-yl]phénoxy]acétate de méthyle (18)

Ce composé (solide vert intense d'aspect métallique) est synthétisé en appliquant la procédure décrite pour la préparation du composé 8.

Masse obtenue : 0,065 g, 0,093 mmol

Rendement : 85%

¹H RMN (400 MHz, Acétone-de) : δ 8,27 - 8,19 (m, 6H), 8,16 - 8,11 (m, 2H), 7,60 - 7,46 (m, 7H), 7,29 (s, 1H), 7,14 - 7,08 (m, 4H), 4,87 (s, 2H), 4,24 - 4,17 (m, 2H), 3,78 (d, *J* = 2,1 Hz, 3H), 3,47 (t, *J* = 6,5 Hz, 2H), 1,98 - 1,89 (m, 2H), 1,83 (q, *J* = 7,4 Hz, 2H).

¹³C RMN (101 MHz, Acétone) : δ 133,16, 131,74, 131,33, 130,48, 130,43, 130,29, 129,60, 129,34, 118,67, 115,76, 115,65, 68,49, 65,69, 52,28, 51,76, 27,13, 26,32.

HRMS : [M+H]⁺ C₃₉H₃₄BF₂N₆O₄ m/z calculé 699,270390 ; mesuré 699,270354 (0,1 ppm)

### Synthèse de 2-[4-[(2Z)-2-[5-[4-[4-(tert-butoxycarbonylamino)butoxy] phényl]-1-difluoroboranyl-3-phényl-pyrrol-2-yl]imino-5-phényl-pyrrol-3-yl]phénoxy]acétate de méthyle (19)

Dans un ballon sous argon à température sont dissous 0,061 g de **18** (0,087 mmol, 1 eq.) et 0,024 g de Boc-ON (Sigma) (0,096 g, 1,1 eq.) dans du THF anhydre. A cette solution sont ajoutés 0,096 mL d'une solution 1M de triméthylphosphine dans le toluène (0,096 mmol, 1,1 eq.). La solution est agitée une nuit à température ambiante. Le lendemain, la solution est contrôlée par CCM et si du produit de départ est encore visible, on ajoute 1 eq. supplémentaire de PMe₃ et de Boc-ON afin de terminer la réaction en une heure. La solution est ensuite lavée avec NaCl sat. et extraite au dichlorométhane. La phase organique est séchée sur MgSO₄, filtrée et concentrée sous vide. Le résidu est purifié par chromatographie sur silice (PE/EA) et 0,061 g de **19** (0.079 mmol, 91%) sont obtenus sous forme d'un solide vert foncé iridescent.

¹H RMN (400 MHz, Acétone-de) δ 8,26 - 8,17 (m, 6H), 8,15 - 8,09 (m, 2H), 7,52 (td, *J* = 9,6, 8,9, 4,8 Hz, 7H), 7,25 (s, 1H), 7,11 - 7,05 (m, 4H), 6,00 (s, 1H), 4,85 (s, 2H), 4,16 (dt, *J* = 6,6, 3,4 Hz, 2H), 3,78 (s, 3H), 3,17 (td, *J* = 7,6, 3,8 Hz, 2H), 1,84 (q, *J* = 7,1 Hz, 2H), 1,69 (p, *J* = 7,4 Hz, 2H), 1,41 (s, 9H).

¹³C RMN (101 MHz, Acétone) δ 169,64, 163,11, 160,51, 160,29, 158,35, 156,72, 146,61, 145,53, 144,69, 143,18, 133,12, 133,07, 133,04, 131,70, 131,27, 130,44, 130,41, 130,37, 130,26, 129,55, 129,31, 126,86, 124,28, 120,79, 118,57, 115,71, 115,62, 78,39, 68,77, 65,66, 52,28, 41,83, 40,75, 28,68, 27,43, 27,21, 24,44.

HRMS : [M+H]⁺ C₄₂H₄₁BF₂N₅O₅ calculé 744,317054, mesuré 744,316900 (0,2 ppm).

### 2. Préparation des composés (C)

### Procédure générale de couplage

Dans un ballon sous argon sont ajoutés du TTHA (TCI Chemicals) (10 eq. si couplage avec une antenne anthraquinone, 3 eq. si couplage avec une antenne azabodipy) dans un mélange acétonitrile distillée/triéthylamine distillée (2/1). La solution est agitée à reflux 2 h jusqu'à ce que le TTHA soit totalement dissous. Dans un second ballon, 1 équivalent d'un composé « antenne-Y-A₂-NHBoc » est déprotégé dans un mélange dichlorométhane/TFA (1/1) pendant 30 minutes à température ambiante. Pendant ce temps, le reflux de la solution de TTHA est stoppé et une fois la solution à température ambiante, le ballon est plongé dans un bain de glace et 0,9 équivalents de HBTU /eq. TTHA dissous dans quelques mL d'acétonitrile sont ajoutés goutte à goutte à la solution. Cette solution est activée pendant 15 minutes. Pendant ce temps, la solution DCM/TFA est évaporée sous vide et le résidu est trituré avec de l'éther diéthylique pour enlever l'excès de TFA. Le surnageant est enlevé à la pipette pasteur et le sel de TFA du composé « antenne-Y-A₂-NH₃⁺ » est séché sous vide. Ce sel est ensuite dissous dans quelques mL d'acétonitrile et quelques gouttes de triéthylamine. Ce mélange est ensuite ajouté goutte à goutte à la solution de TTHA activé. On laisse la réaction remonter à température ambiante et on l'agite toute la nuit. Le lendemain, la réaction est arrêtée par ajout d'eau distillée. On effectue un lavage à l'acétate d'éthyle et le produit est extrait avec de l'eau distillée. Les phases aqueuses sont collectées, concentrées sous vide et le résidu est ensuite lyophilisé une nuit. Par la suite, le résidu est purifié par chromatographie en phase inverse avec un gradient eau/MeOH et éventuellement 0,1% TFA. Les fractions collectées peuvent ensuite être dissoutes dans un mélange eau/MeOH/triéthylamine et on peut effectuer une résine échangeuse d'ions (IRA-400(Cl)) de façon à récupérer le conjugué TTHA-A₂-Y-antenne sous sa forme acide avec une élution MeOH/eau/acide formique.

### Synthèse de L1 : acide 3-(2-((2-((4-((2-aminoéthyl)amino)-5,8-dihydroxy-9,10-dioxo-9,10-dihydroanthracen-1-yl)amino)éthyl)amino)-2-oxoéthyl)-6,9,12-tris(carboxyméthyl)-3,6,9,12-tétraazatétradécane-1,14-dioique

Synthétisé selon la procédure générale de couplage à partir de l'anthraquinone 1,4-Bis[Boc-aminoethylamino]-5,8-dihydroxyanthracene-9,10-dione (comme décrit dans l'article de Sylvain Routier, Nicole Cotelle, Jean-Pierre Catteau, Jean-Luc Bernier, Michael J. Waring, Jean-Francois Riou and Christian Bailly, Bioorganic & Medicinal Chemistry, Vol. 4, No. 8, pp 1185-1196, 1996)

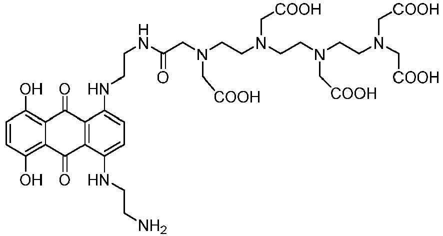

Rendement : 60%, HRMS (ESI) pour C₃₆H₄₈N₈O₁₅ calculé 832,3239, [M+H]⁺ : 833,3301 C₃₆H₄₉N₈O₁₅ (-1,3 ppm), [M+2H]²⁺ 417,1697 C₃₆H₅₀N₈O₁₅ (-1,0 ppm).

¹H RMN (250 MHz, Deuterium Oxide) δ 8,30 (s, 2H), 6,68 (m, 2H) 4,00-3,80 (m, 4H), 3,70-3,25 (m, 30H).

### Synthèse de l'acide 3,6,9-tris(carboxyméthyl)-12-(2-((2-((4-hydroxy-9,10-dioxo-9,10-dihydroanthracen-1-yl)amino)éthyl)amino)-2-oxoéthyl)-3,6,9,12-tétraazatétradécane-1,14-dioique

Synthétisé selon la procédure générale de couplage à partir de l'anthraquinone 1-[(Boc-aminoethyl)amino]-4-hydroxyanthracene-9,10-dione (comme décrit dans l'article de Sylvain Routier, Nicole Cotelle, Jean-Pierre Catteau, Jean-Luc Bernier, Michael J. Waring, Jean-Francois Riou and Christian Bailly, Bioorganic & Medicinal Chemistry, Vol. 4, No. 8, pp 1185-1196, 1996).

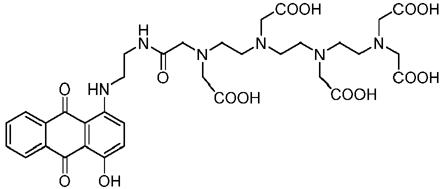

Rendement : 60%. HRMS (ESI) pour C₃₄H₄₂N₆O₁₄ calculé 758,2759, [M+H]⁺ : 759,283160 C₃₄H₄₃N₆O₁₄ 0 ppm), [M+2H]²⁺ 380,145794 C₃₄H₄₄N₆O₁₄ (1,5 ppm).

¹H RMN (250 MHz, Methanol-*d*₄) δ 8,29 - 8,16 (m, 2H), 7,86 - 7,69 (m, 2H), 7,45 (d, *J* = 9,6 Hz, 1H), 7,21 (d, *J* = 9,5 Hz, 1H), 3,85 - 3,70 (m, 3H), 3,58 (m, 3H), 3,46 (m, 2H), 3,20 (m, 14H).

¹³C RMN (63 MHz, MeOD) δ 188,55, 183,08, 170,80, 157,72, 136,49, 135,39, 133,82, 129,80, 127,77, 127,13, 125,89, 118,16, 114,55, 109,69, 68,85, 42,57, 30,68, 26,48, 9,16.

### Synthèse de l'acide 2-[2-[2-[bis(carboxyméthyl)amino]éthyl-(carboxy-méthyl)amino]éthyl-[2-[carboxyméthyl-[2-[2-[[2-[4-[(2Z)-2-[1-difluoroboranyl-3-(4-méthoxyphényl)-5-phényl-pyrrol-2-yl]imino-5-phényl-pyrrol-3-yl]phénoxy] acétyl]amino]éthylamino]-2-oxo-éthyl]amino]éthyl]amino]acétique

Synthétisé selon la procédure générale de couplage à partir de l'azabodipy **10** (comme décrit dans l'article de Sylvain Routier, Nicole Cotelle, Jean-Pierre Catteau, Jean-Luc Bernier, Michael J. Waring, Jean-Francois Riou and Christian Bailly, Bioorganic & Medicinal Chemistry, Vol. 4, No. 8, pp 1185-1196, 1996).

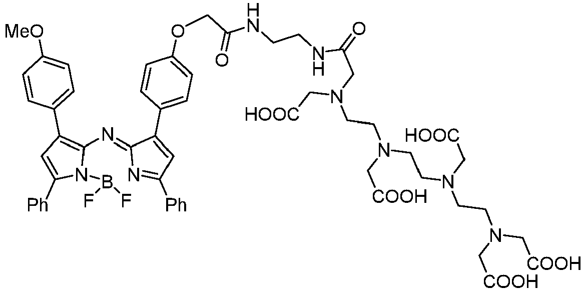

Rendement : 70%

¹H RMN (250 MHz, Methanol-*d*₄) δ 8,19 - 7,99 (m, 8H), 7,67 -7,45 (m, 6H), 7,15 (m, 4H), 7,06 (s, 1H), 7,02 (s, 1H), 4,62 (s, 2H), 3,90-3,80 (m, 15H), 3,50-3,35 (m, 16H).

### Synthèse de l'acide 2-[2-[2-[bis(carboxyméthyl)amino]éthyl-(carboxy-méthyl)amino]éthyl-[2-[carboxyméthyl-[2-[4-[4-[1-difluoroboranyl-5-[(Z)-[3-[4-(2-méthoxy-2-oxo-éthoxy)phényl]-5-phényl-pyrrol-2-ylidène]amino]-4-phényl-pyrrol-2-yl]phénoxy]butylamino]-2-oxo-éthyl]amino]éthyl]amino]acétique

Synthétisé selon la procédure générale de couplage à partir de l'azabodipy **19** (comme décrit dans l'article de Sylvain Routier, Nicole Cotelle, Jean-Pierre Catteau, Jean-Luc Bernier, Michael J. Waring, Jean-Francois Riou and Christian Bailly, Bioorganic & Medicinal Chemistry, Vol. 4, No. 8, pp 1185-1196, 1996).

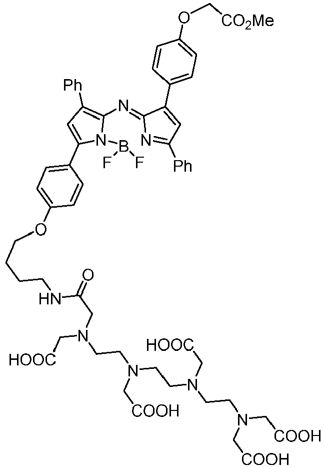

Rendement : 75%

¹H RMN (250 MHz, MeOD) δ 8,18-8,15 (m, 2H), 7,95- 7,9 (m, 3H), 7,86- 7,80 (m, 3H), 7,72-7,67 (m, 2H), 7,65- 7,58 (m, 7H), 7,50-7,30 (m, 3H), 4,64 (s, 2H), 3,61-3,55 (m, 9H), 3,50-3,40 (m, 16H),3,30-3,10 (m, 6H), 1,29- 1,18 (m, 4H).

### Exemple 2 : Synthèse d'un complexe selon l'invention

Le complexe de Nd³⁺ a été synthétisé in situ par le mélange en rapport 1 :1 d'une solution de L1 (comme détaillé ci-dessus), avec une solution de nitrate de néodyme(III) dans un tampon HEPES à pH = 7,5.

### Exemple 3 : Propriétés des complexes selon l'invention

### 1. Propriétés photophysiques

Le ligand L1 et le complexe L1Nd³⁺ (exemple 2) possèdent tous deux des bandes d'absorption larges qui s'étendent sur une gamme de longueur d'onde possédant des valeurs aussi élevées que 750 nm avec des maxima centrés à ~ 615 et ~ 670 nm et des coefficients d'absorption molaires allant jusqu'à 5•10³ M⁻¹ cm⁻¹. Lors de l'excitation des bandes d'absorption à basse énergie localisées sur L1 et sur le complexe L1Nd³⁺, il est possible d'observer des signaux d'émission sous forme de bandes larges provenant du ligand chromophore centrées à ~ 690 nm avec un épaulement à ~ 740 nm. Le complexe L1Nd³⁺, lorsqu'il est excité à une longueur d'onde de 650 nm, produit une bande d'émission étroite avec un maximum à 1 064 nm qui est caractéristique de la structure électronique du Nd³⁺ en résultant de la transition ⁴F_{3/2} →⁴I_{11/2}. Les spectres d'excitation enregistrés en observant soit le signal d'émission du ligand centré à 740 nm, soit le signal d'émission étroit du Nd³⁺ centré à 1 064 nm se superposent aux spectres d'absorption indiquant ainsi que la sensibilisation du cation lanthanide s'opère en utilisant les niveaux électroniques du chromophore dérivé de l'anthraquinone du ligand L1. En conséquence, le ligand L1 et le complexe L1Nd³⁺ possèdent tous deux des longueurs d'onde d'excitation et d'émission incluses dans le domaine de la fenêtre de diagnostic biologique (Figure 1).

Les paramètres quantitatifs de luminescence (temps de vie de luminescence (τ) et rendements quantiques de luminescence centrée sur L1 - (Q_{L}) et sur Nd³⁺-(*Q*_{Nd})) déterminés sur le complexe L1Nd³⁺sont résumés dans le tableau 3.1.

Des valeurs de temps de vie r relativement courtes (dans la gamme de la nanoseconde) et des rendements quantiques *Q*_{Nd} faibles ont été mesurés pour l'émission proche-infrarouge du Nd³⁺ en comparaison aux valeurs correspondantes les plus élevées qui ont été publiées à ce jour (J.-C.G. Bünzli, S.V. Eliseeva, Photophysics of lanthanoid coordination compounds, in: V.W.-W. Yam (Ed.) Comprehensive Inorganic Chemistry II, Elsevier B.V., Amsterdam, 2013, pp. 339-398 ; et J.-C.G. Bünzli, On the design of highly luminescent lanthanide complexes, Coordination Chemistry Reviews, 293-294 (2015) 19-47). L'analyse des temps de vie mesurés sur le complexe L1Nd³⁺ dans H₂O et D₂O révèle qu'aucune molécule d'eau n'est liés directement aux cations lanthanide ce qui constitue une indication forte du bon niveau de protection qui est fourni par l'entité de coordination TTHA.

Le rendement quantique de l'émission provenant du ligand est plus faible d'un rapport de 30% pour le complexe de Nd³⁺ par rapport au ligand L1. Ce résultat est révélateur de la présence d'un transfert d'énergie entre le chromophore anthraquinone et le cation Nd³⁺. Il est important de souligner que les positions des bandes d'émission étroites provenant du Nd³⁺ ne sont pas affectées par les changements de microenvironnements ce qui constitue un avantage majeur par rapport au fluorophore commercial « vert d'indocyanine » qui possède une longueur d'onde d'excitation similaire (Fernandez-Fernandez, R. Manchanda, T. Lei, D.A. Carvajal, Y. Tang, S.Z. Raza Kazmi, A.J. McGoron, Comparative study of the optical and heat generation properties of IR820 and indocyanine green, Molecular imaging, 11 (2012) 99 et S. Mordon, J.M. Devoisselle, S. Soulie-Begu, T. Desmettre, Indocyanine green: physicochemical factors affecting its fluorescencein vivo, Microvascular Research, 55 (1998) 146-152).

**Tableau 3.1. Propriétés photophysiques du ligand L1 et du complexe L1Nd³⁺ (100 µM, pH = 7.5, mesurées à température ambiante).^{a}**

| **Composé** | ***τ*(ns)*^{b}*** | | ***q*(H₂O)*^{c}*** | ***Q*_{Nd}(%)*^{d}*** | | ***Q*_{L}(%)*^{e}*** |
|---|---|---|---|---|---|---|
| | H₂O | D₂O | | H₂O | D₂O | |
| **L1Nd³⁺** | 148(2) | 389(1) | 0,1 | 1,80(3)·10⁻³ | 7,9(2)·10⁻³ | 0,177(8) |
| **L1** | - | - | - | - | - | 0,257(5) |

| | | | | | | |
|---|---|---|---|---|---|---|
| *^{a}* Les valeurs de 2*σ* sont indiquées entre parenthèses. Erreurs expérimentales: *τ*, ±2%, *Q*, ±10%. *^{b}* Obtenu sous excitation à 680 nm. *^{c}* Le nombre de = molécules d'eau coordinées au cation lanthanide (*q*) a été calculé en utilisant les équations: ${q}_{Nd} = 130 \times \left(\frac{1}{{τ}_{H 2 O}}-\frac{1}{{τ}_{D 2 O}}\right) - 0.4$. L'erreur estimée est de ±0.2 (S. Faulkner, A. Beeby, M.-C. Carrié, A. Dadabhoy, A.M. Kenwright, P.G. Sammes, Time-resolved near-IR luminescence from ytterbium and neodymium complexes of the Lehn cryptand, Inorganic Chemistry Communications, 4 (2001) 187-190). *^{d}* obtenue avec une longueur d'onde d'excitation de 650 nm, rendement quantique partiel mesuré pour la transition ⁴F_{3/2}→⁴I_{11/2}. *^{e}* Sous excitation à 620 nm, le signal d'émission a été collecté sur le domaine spectral de 640-850 nm. | | | | | | |

Les photostabilités de L1 et L1Nd³⁺ ont été étudiées sous illumination continue à 630 nm (Figure 2). Il a été démontré que la présence du cation lanthanide (Nd³⁺) améliore de manière significative la photostabilité. Ainsi, les intensités lumineuses émises à partir du ligand (*λ*ₑₘ=740 nm) et du lanthanide Nd³⁺ (*λ*ₑₘ= 1064 nm) au sein du complexe ne diminuent pas après 3h d'illumination continue alors que l'émission du ligand libre perd ~70% de son intensité initiale sous conditions expérimentales identiques. Ces résultats peuvent être expliqués en première hypothèse par la concentration de l'énergie provenant de de l'état triplet de L1 dans le cas du complexe L1Nd³⁺.

### Tests de Cytotoxicité

Afin de déterminer la cytotoxicité du complexe L1Nd³⁺ dans des cellules vivantes, des tests de type "Alamar Blue" ont été effectués sur la ligne cellulaire HeLa. Après 24 h d'incubation avec le complexe L1Nd³⁺, nous avons observé un taux de viabilité de 77% à une concentration de 12 µM. Pour cette raison, une concentration de travail de 5 µM correspondant à un taux de viabilité cellulaire de 90% a été choisie pour les expériences de microscopie. Des temps d'incubation plus courts (5h) ont permis de préserver la viabilité des cellules HeLa sans que des effets résultant de la présence du complexe L1Nd³⁺ puissent être détectés jusqu'à des concentrations aussi élevées que 240 µM (Figure 3).

### Microscopie Confocale

L'internalisation du complexe L1Nd³⁺ dans des cellules vivantes HeLa a été confirmée par des expériences de microscopie confocales sur des échantillons obtenus après 3h d'incubation avec une solution de L1Nd³⁺ de 5 µM. Lorsque l'on observe ces cellules HeLa incubées en utilisant une excitation laser à 633 nm et en sélectionnant des coupes optiques d'épaisseurs de 1 µm selon le plan vertical, l'émission provenant du dérivé d'anthraquinone a pu être détectée sur une gamme de longueur d'onde de 650 à 750 nm (Figure 4). La distribution intracellulaire du signal de luminescence suggère une localisation avec les lysosomes. L'absence de signaux d'autofluorescence pour ces longueurs d'onde confirme que l'imagerie opérée dans la fenêtre de diagnostique biologique permet d'améliorer de manière significative le rapport signal sur bruit de la mesure et la sensibilité de détection.

Lorsque les cellules HeLa ont été incubées avec des concentrations plus élevées de complexe L1Nd³⁺ (50 µM), le signal de luminescence a pu être détecté non seulement dans les lysosomes mais également dans les nucléosomes (Figure 5).

### Cytométrie de flux

En complément des expériences de microscopie confocale, l'internalisation du complexe L1Nd³⁺ a été confirmée de manière quantitative par une analyse en cytométrie de flux conduite sur un échantillon de 10⁴ cellules sous excitation à 633 nm et en analysant l'émission provenant du chromophore dérivé de l'anthraquinone localisée sur le ligand chromophore (Figure 6).

Le mécanisme d'incorporation cellulaire a été analysé par inhibition des transports actifs, qui sont dépendants de l'énergie (incubation avec de l'azide de sodium NaN₃) et passifs (incubation à 4°C). En raison de l'augmentation de la rigidité de la membrane lors de l'incubation at 4°C, en plus de l'inhibition du transport passif, ceux qui sont actifs sont également désactivés. Les résultats obtenus permettent de conclure sans ambigüité que le complexe L1Nd³⁺ est incorporé principalement par le biais d'un mécanisme de transport passif puisque 69±10% de l'incubation de L1Nd³⁺ a été bloqué par l'incubation at 4°C alors que 11± 5% de complexe L1Nd³⁺ ont étés transportés par un processus actif.

### Microscopie d'épifluorescence

En dépit du rendement quantique faible de L1Nd³⁺, il a été possible d'effectuer des mesures d'imagerie proche-infrarouge avec une bonne sensibilité dans des cellules Hela à l'aide de cet agent d'imagerie. Un signal proche-infrarouge intense (sélectionné au moyen d'un filtre passe bas à 805 nm) a pu être observé pour le complexe L1Nd³⁺ dans ces cellules HeLa en excitant L1Nd³⁺ en utilisant une source d'excitation conventionnelle (lampe Xénon, filtre à 655 nm axant une bande passante des 40 nm) (Figure 7C, haut). En raison de l'absence d'autofluorescence dans le proche-infrarouge et en utilisant le même montage expérimental pour les cellules de contrôle par rapport aux cellules incubées avec le complexe L1Nd³⁺ (Figure 7C, bas), nous avons confirmé un rapport signal sur bruit et une sensibilité de détection fortement améliorés pour des agents d'imagerie de fluorescence possédant des longueurs d'onde d'excitation et d'émission situées dans la fenêtre de diagnostique biologique.

Dans le cas de l'utilisation de la sonde commerciale visible utilisée pour le marquage spécifique des lysosomes, le lysotracker jaune, une autofluorescence peut être détectée étant générée par des biomolécules puisque ces dernières possèdent une forte absorption et émission dans cette région (figure 7B, en bas). Cependant, la localisation du complexe L1Nd³⁺ à l'intérieur des lysosomes a été confirmée par une expérience de microscopie d'épifluorescence lorsque le lysotracker jaune a été localisé au même endroit que le complexe L1Nd³⁺ (figure 7d, en haut).

En outre, il a été observé avec des expériences de microscopie confocale que lorsque les cellules HeLa sont incubées avec une concentration plus élevée de complexe L1Nd³ (50 µM), qu'une localisation peut également obtenue dans les nucléosomes où un signal proche infrarouge intense a pu être détecté (figure 8).

### Test de photoblanchiment menée par microscopie d'épifluorescence

Ain de comparer la photostabilité du complexe L1Nd³⁺ avec celle d'un rapporteur disponible commercialement, un test de photoblanchiment a été effectué par comparaison avec le «LysoTracker Deep Red » avec un suivi par microscopie d'épifluorescence. Un photoblanchiment important a été observé dans le cas du rapporteur "LysoTracker Deep Red" après 20s d'illumination continue à une lumière sélectionnée par un filtre à 655 nm (de bande passante de 40nm) (Figure 9, haut). Par contre, l'intensité de l'émission provenant de l'anthraquinone dans le complexe L1Nd³⁺ est restée constante durant 95s en utilisant les mêmes conditions expérimentales (sélection de la longueur d'onde par un filtre de 655 nm de bande passante de 40 nm pour l'excitation et 716 nm de bande passante de 40 nm pour l'émission (Figure 9, en bas).

Un signal d'émission constant collecté au travers d'un filtre coupe bas (805 nm, Figure 10) a pu être observé révélant une photostabilité très satisfaisante a été mesurée jusqu'à 495 secondes pour le complexe L1Nd³⁺ sous illumination continue au moyen de lumière sélectionnée par un filtre à 655 nm de bande passante de 40 nm.

### Macroscopie d'épifluorescence : détection de l'émission proche-infrarouge provenant du complexe L1Nd³⁺ dans/au travers de différents échantillons biologiques

Afin de démontrer d'une manière complémentaire les avantages de l'utilisation des nouveaux agents d'imagerie selon l'invention qui possèdent des longueurs d'onde d'excitation et d'émission dans le domaine de la fenêtre de diagnostique biologique et tout particulièrement la pénétration des photons à de plus grandes profondeurs, des expériences ont été conduites sur des tissus biologiques d'origine animale et d'épaisseurs variées en utilisant le montage expérimental suivant : un capillaire en verre contenant une solution de 200 µM de L1Nd³⁺ a été placée sous les différents échantillons de tissus (Figure 11, en haut) et excitée par le biais d'un laser à colorant pulsé émettant une lumière de longueur d'onde de 685 nm. Un signal intense a pu être collecté en utilisant un filtre coupe bas à 785 nm (Figure 11, bas). Cette expérience a permis de mettre en évidence que les photons proche-infrarouge provenant du complexe L1Nd³⁺ peuvent être détectés au travers de tissus de porc d'une épaisseur de 3mm, de foie de poulet (2 mm), de bœuf (1,6mm) ou de peau de poulet (0,6mm). Les tests sur des tissus de natures différentes ont permis d'évaluer les différences absorption des tissus et les différentes diffractions de lumière qui sont deux paramètres liés à la profondeur de pénétration de photons et qui influencent la résolution des images collectées. De plus, une différence d'autofluorescence générée en fonction de la nature des tissus a été observée.

Etant donné que les composants du sang, en particulier l'hémoglobine font partie des plus grands absorbeurs de lumière et des plus grand émetteurs d'autofluorescence, ces derniers peuvent être responsables d'une très grande perte de signal et peuvent conduire à une très grande diminution du rapport signal sur bruit et de sensibilité de mesure (A. Taruttis, V. Ntziachristos, Translational optical imaging, American journal of roentgenology American Roentgen Ray Society, 199 (2012) 263-271). Il a été démontré ici qu'en utilisant des longueurs d'onde d'excitation et d'émission comprises dans la fenêtre de diagnostic biologique, qu'il est possible de minimiser de manière efficace les effets indésirables de l'autofluorescence. Ainsi, il a été possible de détecter un signal proche infrarouge intense provenant de L1Nd³⁺ à une concentration de 200 µM dans le sang.

## Revendications

1. Complexe comprenant au moins un lanthanide (Ln) et au moins un composé (C) répondant à la formule (II) suivante : 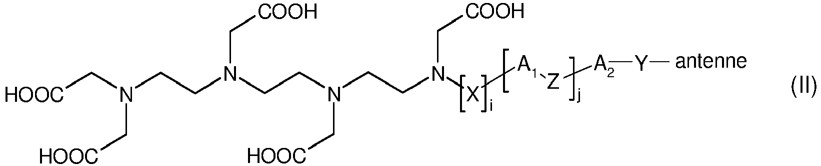 dans laquelle :
- i est 0 ;
- j est un nombre entier compris entre 1 et 3 ;
- X est choisi parmi les fonctions -NH-NH-CO-, thioester, ester et amide,
- A₁ et A₂ sont choisis, indépendamment l'un de l'autre, parmi les radicaux (cyclo)alkylènes, linéaires ou ramifiés, comprenant de 1 à 12 atomes de carbone ;
- Z est -C(=O)-NH- ou -NH-C(=O)- ; et
- Y est choisi parmi les groupes -O-, -NH-, -S-, alkylène, amide, ester, triazole, amine, urée, thiourée, imine, oxyme, hydrazone, sulfonamide, carbamate, amidine, phosphoramidate, disulfure et sulfonyle ;
ledit motif de formule (I) étant relié de façon covalente à au moins une antenne qui absorbe à une longueur d'onde allant de 500 nm à 900 nm, ladite antenne étant choisie dans le groupe constitué des anthraquinones, des cyanines, notamment des cyanines 5 et des cyanines 7, des aza-BODIPY, des pérylènediimides et des sels de phénothiazine.

2. Complexe selon la revendication 1, dans lequel le composé (C) répond à la formule (IV) suivante : 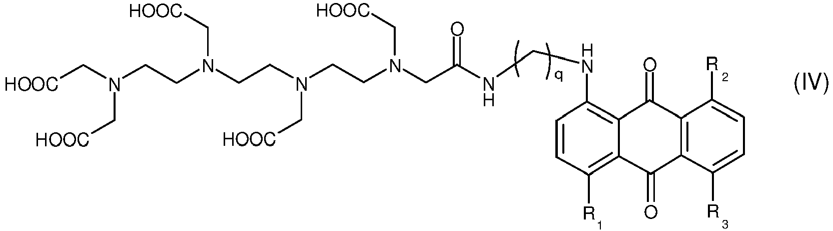 dans laquelle :
- q étant un nombre entier compris entre 1 et 6 ;
- R₁ représentant :
. H ;
. un groupe -NH-(CH₂)ₙ-NH₂, n représentant un nombre entier compris entre 1 et 6 ;
. un groupe -NH-(CH₂)ₙ-R_{α}, n étant tel que défini ci-dessus et Rₐ représentant un groupe choisi parmi les halogènes, les acides carboxyliques, les esters de succinimide, les esters de tétrafluorophényle, les azotures d'acyle, les anhydrides, les halogénures d'acide, les acrylamides, les alcools, les amines, les alcynes, les cyclooctines, les aminooxyacétamides, les azotures, les imidoesters, les esters de sulfonate, les halogéno-acétamides, les halogénures d'alkyle, les halogénures de sulfonyle, les hydrazines, les hydrazides, les isocyanates, les isothiocyanates, les tétrazines et les maléimides ;
. un groupe (C₁-C₆)alkyle ;
. un groupe ORₐ, Rₐ représentant H ou un groupe (C₁-C₆)alkyle ;
. un groupe NRₐR_{b}, Rₐ et R_{b} représentant, identiques ou différents, H ou un groupe (C₁-C₆)alkyle ; et
- R₂ et R₃, identiques ou différents, représentant :
. H ;
. un groupe (C₁-C₆)alkyle ;
. un groupe ORₐ, Rₐ représentant H ou un groupe choisi parmi les groupes (C₁-C₆)alkyle, (C₆-C₁₀)aryle et (C₅-C₁₀)hétéroaryle ;
. un groupe -NH-(CH₂)ₙ-R_{α}, n et R_{α} étant tels que définis ci-dessus ; et
. un groupe NRₐR_{b}, Rₐ et R_{b} représentant, identiques ou différents, H ou un groupe choisi parmi les groupes (C₁-C₆)alkyle, (C₆-C₁₀)aryle et (C₅-C₁₀)hétéroaryle.

3. Complexe selon la revendication 1, dans lequel le composé (C) répond à la formule (VI) suivante : 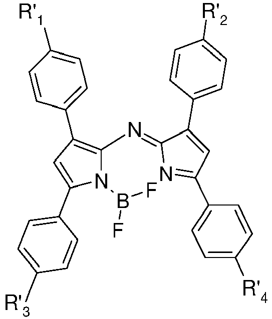 dans laquelle :
- R'₁ est H ou est choisi parmi les groupes (C₁-C₆)alkyle, (C₁-C₆)alcoxy, NRₐR_{b}, Rₐ et R_{b}, identiques ou différents, représentant H ou un groupe (C₁-C₆)alkyle ;
- R'₂ est :
* soit un groupe -O-A₃-COORₐ, A₃ représentant un radical (C₁-C₆)alkylène et Rₐ représentant H ou un groupe (C₁-C₆)alkyle ;
* soit un groupe de formule (VI-1) suivante : 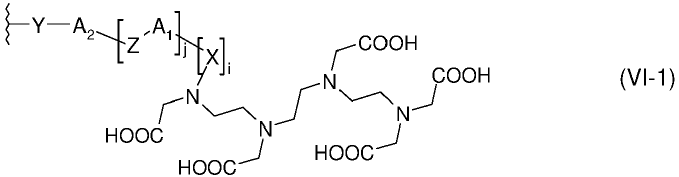 dans laquelle i, j, X, A₁, A₂, Y et Z sont tels que définis dans la revendication 1 ;
- R'₃ est H ou un groupe de formule (VI-1) susmentionnée ;
- R'₄ est H ou est choisi parmi les groupes (C₁-C₆)alkyle, (C₁-C₆)alcoxy, NRₐR_{b}, Rₐ et R_{b}, identiques ou différents, représentant H ou un groupe (C₁-C₆)alkyle ;
sous réserve que lorsque R'₃=H alors R'₂ est un groupe de formule (VI-1) et lorsque R'₂= -O-A₃-COORₐ alors R'₃ est un groupe de formule (VI-1).

4. Complexe selon la revendication 3, dans lequel le composé (C) répond à la formule (VII) suivante : 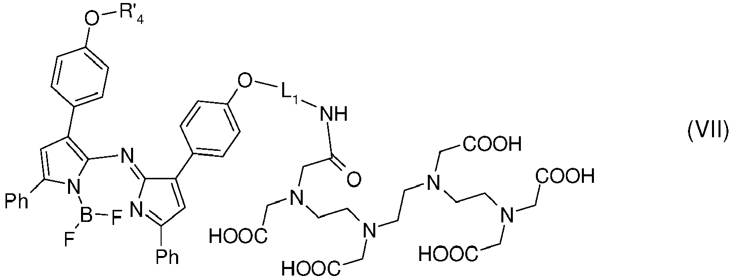 dans laquelle :
- L₁ représente un linker choisi parmi :
. les radicaux -(CH₂)ₘ-C(=O)-NH-(CH₂)ₚ-, m et p représentant un nombre entier compris entre 1 et 4,
- R'₄ représente un groupe (C₁-C₆)alkyle.

5. Complexe selon la revendication 3, dans lequel le composé (C) répond à la formule (VIII) suivante : 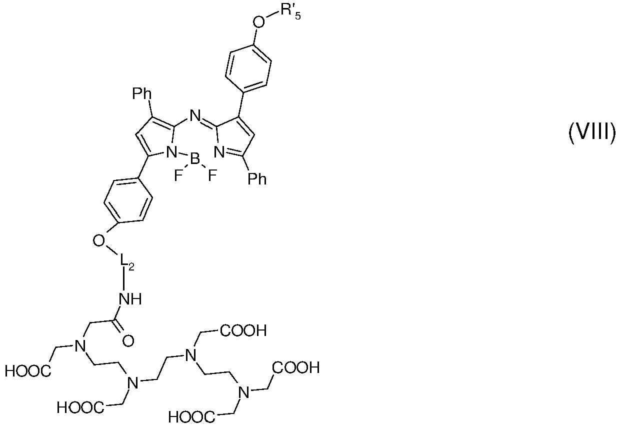 dans laquelle :
- L₂ représente un radical (C₁-C₆)alkyklène, et
- R'₅ représente un groupe (C₁-C₆)alkyle, substitué par un groupe COORₐ, Rₐ représentant H ou un groupe (C₁-C₆)alkyle.

6. Complexe selon l'une quelconque des revendications 1 à 5, dans lequel le composé (C) répond à l'une des formules suivantes :

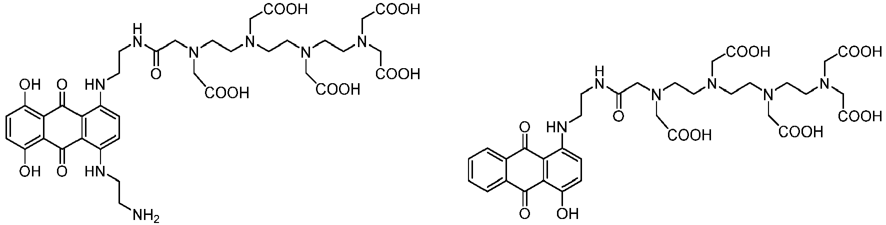

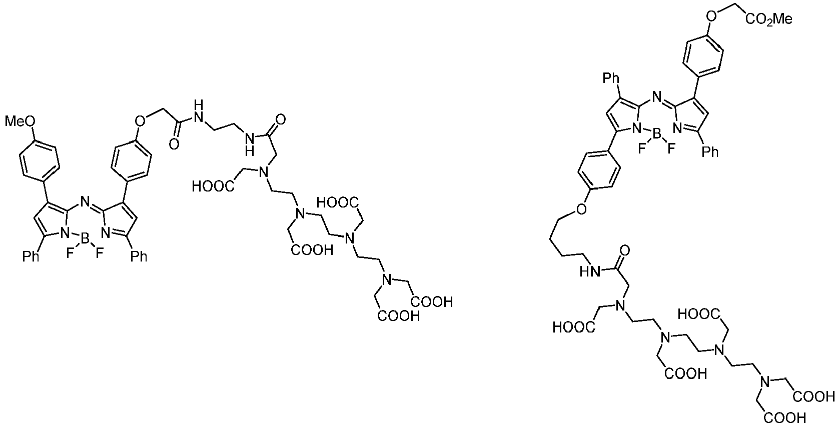

7. Complexe selon l'une quelconque des revendications 1 à 6, dans lequel le lanthanide est choisi dans le groupe constitué de Yb, Nd, Ho, Tm, Sm, Dy, Eu, Pr et Er.

8. Utilisation d'un complexe selon l'une quelconque des revendications 1 à 7, comme chromophore fluorescent.

## Patentansprüche

1. Komplex, umfassend mindestens ein Lanthanid (Ln) und mindestens eine Verbindung (C), die der folgenden Formel (II) entspricht: 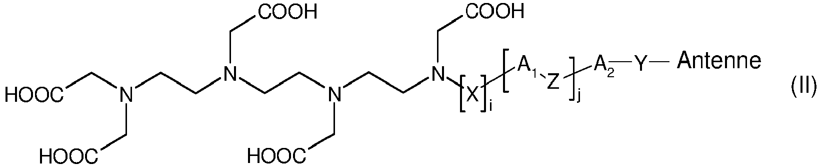 wobei:
- i 0 ist;
- j eine ganze Zahl zwischen 1 und 3 ist;
- X ausgewählt ist aus den Funktionen -NH-NH-CO-, Thioester, Ester und Amid,
- A₁ und A₂ unabhängig ausgewählt sind aus linearen oder verzweigten (Cyclo)alkylenradikalen, umfassend 1 bis 12 Kohlenstoffatome;
- Z -C(=O)-NH- oder -NH-C(=O)- ist; und
- Y ausgewählt ist aus den Gruppen -O-, -NH-, -S-, Alkylen, Amid, Ester, Triazol, Amin, Harnstoff, Thioharnstoff, Imin, Oxim, Hydrazon, Sulfonamid, Carbamat, Amidin, Phosphoramidat, Disulfid und Sulfonyl;
wobei das Motiv von Formel (I) kovalent mit mindestens einer Antenne verbunden ist, die bei einer Wellenlänge von 500 nm bis 900 nm absorbiert, wobei die Antenne ausgewählt ist aus der Gruppe, bestehend aus Anthrachinonen, Cyaninen, insbesondere Cyaninen 5 und Cyaninen 7, aza-BODIPY, Perylendiimiden und Phenothiazinsalzen.

2. Komplex nach Anspruch 1, wobei die Verbindung (C) der folgenden Formel (IV) entspricht: 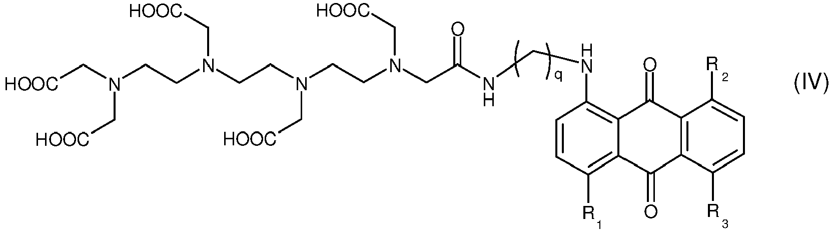 wobei:
- q eine ganze Zahl zwischen 1 und 6 ist;
- Ri Folgendes darstellt:
. H;
. eine Gruppe -NH-(CH₂)ₙ-NH₂, wobei n eine ganze Zahl zwischen 1 und 6 darstellt;
. eine Gruppe -NH-(CH₂)ₙ-Rₐ, wobei n wie oben definiert ist und R_{α} eine Gruppe darstellt, die ausgewählt ist aus Halogenen, Carbonsäuren, Succinimidestern, Tetrafluorphenylestern, Acylaziden, Anhydriden, Säurehalogeniden, Acrylamiden, Alkoholen, Aminen, Alkinen, Cyclooctinen, Aminooxyacetamiden, Aziden, Imidoestern, Sulfonatestern, Halogenacetamiden, Alkylhalogeniden, Sulfonylhalogeniden, Hydrazinen, Hydraziden, Isocyanaten, Isothiocyanaten, Tetrazinen und Maleimiden;
. eine Gruppe (C₁-C₆)-Alkyl;
. eine Gruppe ORₐ, wobei Rₐ H oder eine Gruppe (C₁-C₆)-Alkyl darstellt;
. eine Gruppe NRₐR_{b}, wobei Ra und Rb gleich oder verschieden sind und H oder eine Gruppe (C₁-C₆)-Alkyl darstellen; und
- R₂ und R3, die gleich oder verschieden sind, Folgendes darstellen:
. H;
. eine Gruppe (C₁-C₆)-Alkyl;
. eine Gruppe ORₐ, wobei RₐH oder eine Gruppe darstellt, ausgewählt aus den Gruppen (C₁-C₆)-Alkyl, (C₆-C₁₀)-Aryl und (C₅-C₁₀)-Heteroaryl;
. eine Gruppe -NH-(CH₂)ₙ-Rₐ, wobei n und R_{α} wie oben definiert sind; und
. eine Gruppe NRₐR_{b}, wobei Rₐ und Rb, die gleich oder verschieden sind, H oder eine Gruppe sind, ausgewählt aus den Gruppen(C₁-C₆)-Alkyl, (C₆-C₁₀)-Aryl und (C₅- C₁₀)--Heteroaryl.

3. Komplex nach Anspruch 1, wobei die Verbindung (C) der folgenden Formel (VI) entspricht: 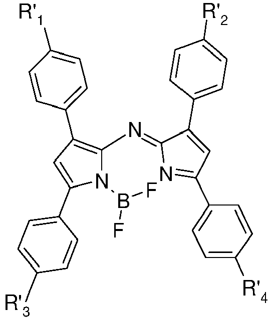 wobei:
- R'₁ H ist oder ausgewählt ist aus den Gruppen (C₁-C₆)-Alkyl, (C₁-C₆)-Alkoxy, NRₐRb, Rₐ und R_{b}, die gleich oder verschieden sind, H oder eine Gruppe (C₁-C₆)-Alkyl sind;
- R'₂ wie folgt ist:
* entweder eine Gruppe -O-A₃-COORₐ, wobei A₃ ein Radikal (C₁-C₆)-Alkylen darstellt und Rₐ H oder eine (C₁-C₆)-Alkyl darstellt;
* oder eine Gruppe von folgender Formel (VI-1): 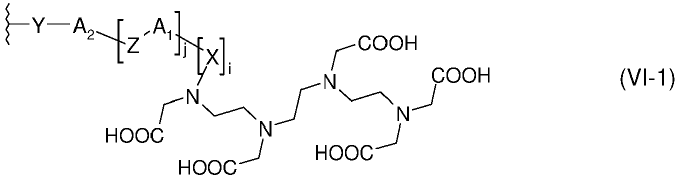 wobei i, j, X, Ai, A₂, Y und Z wie definiert in Anspruch 1 sind;
- R'₃ H oder eine Gruppe der oben erwähnten Formel (VI-1) ist;
- R'4 H ist oder ausgewählt ist aus den Gruppen (C₁-C₆)-Alkyl, (C₁-C₆)Alkoxy, NRₐRb, wobei Ra und Rb, die gleich oder verschieden sind, H oder (C₁-C₆)-Alkyl darstellen;
mit der Maßgabe, dass, wenn R'₃=H, dann R'₂ eine Gruppe von Formel (VI-1) ist, und wenn R'₂= -O-A₃-COORₐ, dann R'₃ eine Gruppe von Formel (VI-1) ist.

4. Komplex nach Anspruch 3, wobei die Verbindung (C) der folgenden Formel (VII) entspricht: 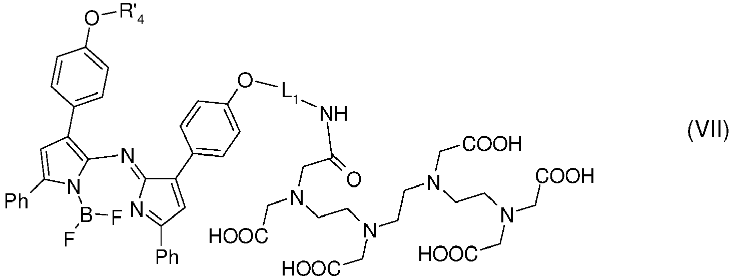 wobei:
- L1 einen Linker darstellt, der ausgewählt ist aus:
. den Radikalen -(CH₂)ₘ-C(=O)-NH-(CH₂)_{P}-, wobei m und p eine ganze Zahl zwischen 1 und 4 darstellen,
- R'4 eine Gruppe (C₁-C₆)-Alkyl darstellt.

5. Komplex nach Anspruch 3, wobei die Verbindung (C) der folgenden Formel (VIII) entspricht: 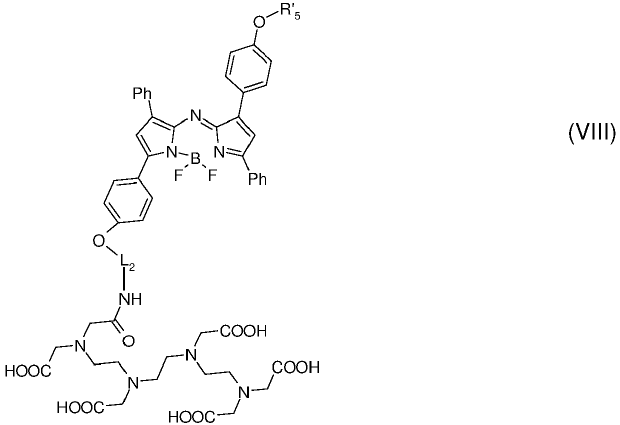 wobei:
- L₂ ein Radikal (C₁-C₆)-Alkylen darstellt und
- R'₅ eine Gruppe (C₁-C₆)-Alkyl darstellt, substituiert mit einer Gruppe COORₐ, wobei Rₐ H oder eine Gruppe ((C₁-C₆)-Alkyl darstellt.

6. Verfahren nach einem der Ansprüche 1 bis 5, wobei die Zusammensetzung (C) mindestens einen der folgenden Formeln entspricht:

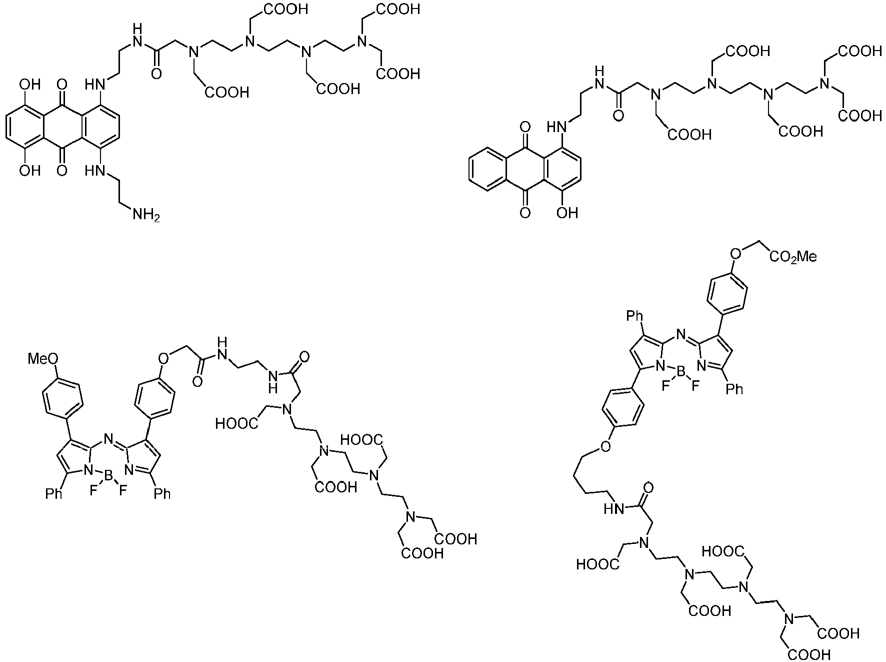

7. Komplex nach einem der Ansprüche 1 bis 6, wobei das Lanthanoid ausgewählt ist aus der Gruppe, bestehend aus Yb, Nd, Ho, Tm, Sm, Dy, Eu, Pr und Er.

8. Verwendung eines Komplexes nach einem der Ansprüche 1 bis 7 als fluoreszierendes Chromophor.

## Claims

1. Complex comprising at least one lanthanide (Ln) and at least one compound (C) having the following formula (II): 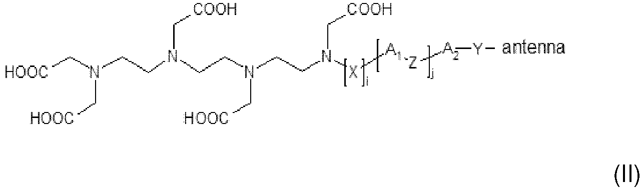 in which:
- i is 0;
- j is an integer between 1 and 3;
- X is chosen from the functions -NH-NH-CO-, thioester, ester and amide,
- A₁ and A₂ are chosen, independently of one another, from linear or branched (cyclo)alkylene radicals comprising from 1 to 12 carbon atoms;
- Z is -C(=O)-NH- or -NH-C(=O)-; and
- Y is selected from -O-, -NH-, -S-, alkylene, amide, ester, triazole, amine, urea, thiourea, imine, oxyme, hydrazone, sulfonamide, carbamate, amidine, phosphoramidate, disulfide and sulfonyl groups;
said unit of formula (I) being connected covalently to at least one antenna which absorbs at a wavelength ranging from 500 nm to 900 nm, said antenna being chosen from the group consisting of: anthraquinones, cyanines, in particular cyanines 5 and cyanines 7, aza-BODIPY, perylenediimides, and phenothiazine salts.

2. Complex according to claim 1, wherein the compound (C) has the following formula (IV): 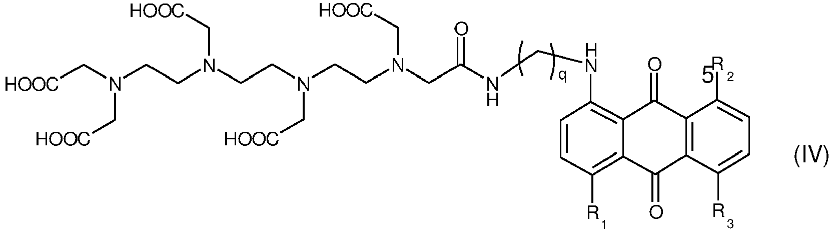 in which:
- q being an integer between 1 and 6;
- R₁ representing:
∘ H;
∘ an -NH-(CH₂)ₙ-NH₂, group, where n is an integer from 1 to 6;
∘ a group -NH-(CH₂)ₙ-Rₐ, n being as defined above and R_{α} representing a group chosen from: halogens, carboxylic acids, succinimide esters, tetrafluorophenyl esters, acyl azides, anhydrides, acid halides, acrylamides, alcohols, amines, alkynes, cyclooctines, aminooxyacetamides, azides, imidoesters, sulfonate esters, haloacetamides, alkyl halides, sulfonyl halides, hydrazines, hydrazides, isocyanates, isothiocyanates, tetrazines, and maleimides;
∘ a (C₁-C₆)alkyl group;
∘ a group ORₐ, Rₐ representing H or a group (C₁-C₆)alkyl;
∘ an NRₐR_{b} group, Rₐ and R_{b} representing, identical or different, H or a (C₁-Ce)alkyl group; and
- R₂ and R₃, identical or different, representing:
∘ H;
∘ a (C₁-C₆)alkyl group;
∘ an ORₐ group, Rₐ being H or a group selected from a (C₁-C₆)alkyl, (C₆-C₁₀)aryl and (C₅-C₁₀)heteroaryl group;
∘ an NH-(CH₂)ₙ-R_{α}, group, n and R_{α} being as defined above; and
∘ an NRₐR_{b} group, Rₐ and R_{b}, identical or different, representing H or a group selected from the (C₁-C₆)alkyl, (C₆-C₁₀)aryl and (C₅-C₁₀)heteroaryl group.

3. Complex according to claim 1, wherein the compound (C) has the following formula (VI): 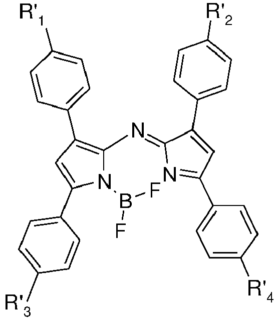 in which:
- R'₁ is H or is selected from the (C₁-C₆)alkyl, (C₁-C₆)alkoxy, and NRₐR_{b} groups, Rₐ and R_{b}, identical or different, representing H or a (C₁-C₆)alkyl group;
- R'₂ is:
∘ an -O-A₃-COORₐ group, A₃ representing a (C₁-C₆)alkylene radical and Rₐ representing H or a (C₁-C₆)alkyl group;
∘ a group of formula (VI-1) below: 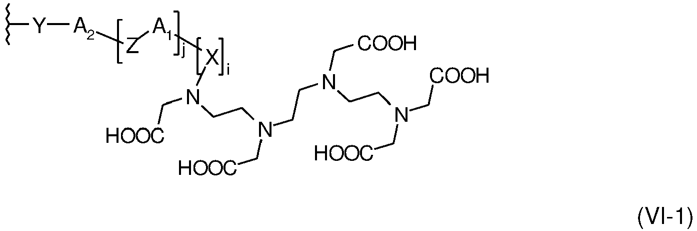
in which i, j, X, A1, A2, Y and Z are as defined in claim 1;
- R'₃ is H or a group of formula (VI-1) above;
- R'₄ is H or is selected from the (C₁-C₆)alkyl, (C₁-C₆)alkoxy, and NRₐR_{b} groups, Rₐ and R_{b}, identical or different, representing H or a (C₁-C₆)alkyl group;
provided that when R'₃ = H then R'₂ is a group of formula (VI-1) and when R'₂ = -O-A₃-COORₐ then R'₃ is a group of formula (VI-1).

4. Complex according to claim 3, wherein the compound (C) has the following formula (VII): 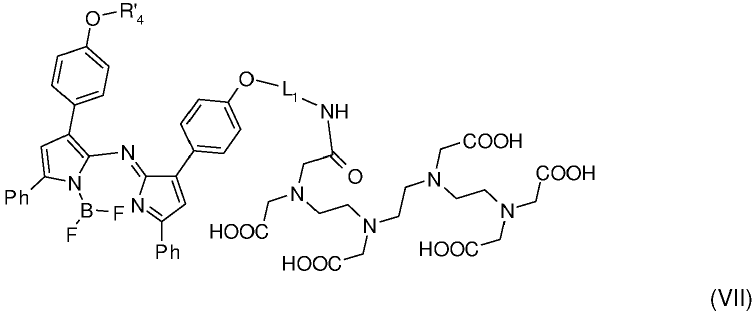 in which:
- L₁ represents a linker chosen from:
o the radicals -(CH₂)ₘ-C(=O)-NH-(CH₂)ₚ-, m and p representing an integer between 1 and 4,
- R'₄ represents a (C₁-C₆)alkyl group.

5. Complex according to claim 3, wherein the compound (C) has the following formula (VIII): 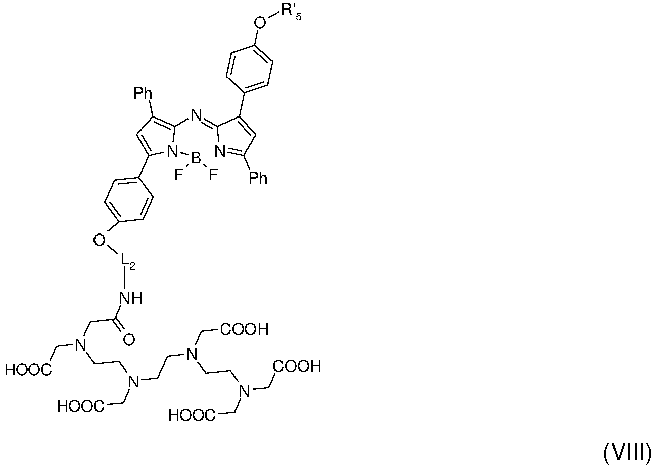 in which:
- L₂ represents a (C₁-C₆)alkylene radical, and
- R'₅ represents a (C₁-C₆)alkyl group, substituted by a COORₐ group, Rₐ representing H or a (C₁-C₆)alkyl group.

6. Complex according to any one of claims 1 to 5, wherein the compound (C) has one of the following formulas:

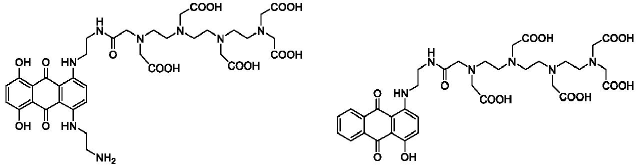

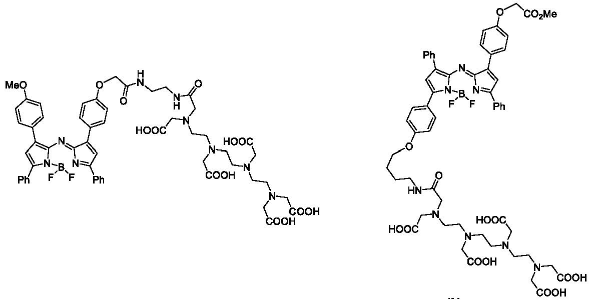

7. Complex according to any one of claims 1 to 9, wherein the lanthanide is selected from the group consisting of: Yb, Nd, Ho, Tm, Sm, Dy, Eu, Pr, and Er.

8. Use of a complex according to any one of claims 1 to 7 as a fluorescent chromophore.
